# EUROPEAN PATENT SPECIFICATION

(11) **EP 1 567 195 B1**
(45) Date of publication and mention of the grant of the patent: **27.10.2010**
(21) Application number: 03775106.2
(22) Date of filing: 26.11.2003
(51) Int. Cl.: A61K 47/48, C08G 83/00

(54) **DENDRIMER CONJUGATES FOR SELECTIVE SOLUBILISATION OF PROTEIN AGGREGATES**
DENDRIMERKONJUGATE FÜR SELEKTIVE LÖSUNG VON PROTEINAGGREGATEN
CONJUGUES DE DENDRIMERES POUR SOLUBILISATION SELECTIVE D'AGREGATS DE PROTEINES

(30) Priority: 26.11.2002 DK 200201828
(43) Date of publication of application: 31.08.2005
(73) Proprietor: UPFRONT CHROMATOGRAPHY A/S, 2100 Copenhagen Ö (DK)
(72) Inventor: HEEGAARD, Peter, DK-2100 Kobenhavn O (DK); BOAS, Ulrik, DK-2200 Kobenhavn N (DK)
(74) Representative: Johansen, Marianne
(86) International application number: PCT/DK2003/000812
(87) International publication number: WO 2004/047869

(56) References cited:
- WO-A-00/72851
- WO-A-01/54736
- WO-A-03/000877
- WO-A1-2004/072153
- WO-A1-2004/072153
- US-A- 4 587 329
- SUPATTAPONE S ET AL: "Branched polyamines cure prion-infected neuroblastoma cells." JOURNAL OF VIROLOGY. APR 2001, vol. 75, no. 7, April 2001 (2001-04), pages 3453-3461, XP002249542 ISSN: 0022-538X
- SUPATTAPONE SURACHAI ET AL: "Elimination of prions by branched polyamines and implications for therapeutics." PROCEEDINGS OF THE NATIONAL ACADEMY OF SCIENCES OF THE UNITED STATES OF AMERICA, vol. 96, no. 25, 7 December 1999 (1999-12-07), pages 14529-14534, XP0002186756 ISSN: 0027-8424
- CALLAHAN M A ET AL: "Reversibility of scrapie-associated prion protein aggregation." THE JOURNAL OF BIOLOGICAL CHEMISTRY. 27 JUL 2001, vol. 276, no. 30, 27 July 2001 (2001-07-27), pages 28022-28028, XP0001094078 ISSN: 0021-9258
- WINKLHOFER K F ET AL: "A sensitive filter retention assay for the detection of PrP<Sc> and the screening of anti-prion compounds" FEBS LETTERS 10 AUG 2001 NETHERLANDS, vol. 503, no. 1, 10 August 2001 (2001-08-10), pages 41-45, XP002249545 ISSN: 0014-5793
- MARSTON ET AL ABELSON J N ET AL: "Solubilization of Protein Aggregates" GUIDE TO PROTEIN PURIFICATION, METHODS IN ENZYMOLOGY, SAN DIEGO, ACADEMIC PRESS, US, vol. VOL. 182, 1990, pages 264-276, XP002900804

## Description

### FIELD OF THE INVENTION

The present invention relates to dendrimer conjugates formed between a dendrimer and a protein solubilizing substance. The dendrimer conjugates are effective in the treatment of protein aggregate related diseases like e.g. prion-related diseases, Alzheimer's etc. The dendrimer conjugates make the protein aggregates more soluble in a reaction medium. The increase in the solubility of the protein aggregates is due to a synergistic effect of the dendrimer conjugate, i.e. a physical mixture of the individual components (dendrimer and protein solubilizing substance) will not increase the solubility to the same extent as the dendrimer conjugate.

### BACKGROUND

Protein aggregates are involved in a number of pathological processes, including prion-related diseases and amyloid-related diseases (Alzheimer's disease, Creutzfeldt-Jakob disease, bovine spongiform encephalopathy, Parkinson's disease, diabetes type II, Huntington's disease and others). These diseases are not very well understood, all are substantially incurable, and all are devastating or fatal. In addition, the prion group of these diseases is highly transmissible and, in one case viz. bovine spongiform encephalopathy, even zoonotic, meaning that it can spread from animals to humans. All the diseases belonging to this group are dependent on the formation of protein aggregates formed upon proteolytic cleavage or upon abnormal folding of normal, naturally-occurring precursor proteins. The transmission of prion diseases is thought to occur through unconventional mechanisms, involving the induction by abnormally-folded prion protein of abnormal and pathogenic folding in normally-folded host prion protein that is normally expressed in the host. In addition, some prion diseases are heritable and are thought to occur through mutations in the prion gene; such mutations facilitating the formation of the pathogenic structural isoform of the prion protein.

One hallmark of the protein aggregates of these diseases is their substantial insolubility in aqueous buffers and their very high protease resistance. Certain methods exist that can be used for solubilisation of protein aggregates e.g. in connection with solubilisation of intracellular protein aggregates often seen in heterologous expression systems (Marston & Hartley, 1990, "Solubilisation of protein aggregates", Meth. Enzymol. 182. 264-276). It is evident from this and other previous work that hydrophobic interactions play a crucial role in stabilising such aggregates in aqueous solutions, and that they can be solubilised by using a number of well-known denaturing solvents and salts, all being characterised by being able to reduce the ordered structure of the water molecules of the solvent (chaotropic effect) thereby decreasing the possibility of hydrophobic interactions. Such salt and solvents include high concentrations of guanidine HCl (5- 8 M) and urea (6-8 M), detergents as SDS and solvents as acetonitrile, isopropanol and other organic solvents. Although it has been know for many years that aggregated PrP can be solubilised with aggregate-solubilising compounds as those described above (Callahan et al., 2001, J. Biol. Chem. 276, 28022-28028) there is still a need to develop solubilising compounds for medical use, i.e. compounds that are not toxic to live cells and organisms. This is needed, as it is known in the prion diseases that it is the aggregates themselves that lead to clinical disease and pathological alterations in the brain of the affected individual. There is also a need to be able to unfold prion protein aggregates in a more reproducible and discriminatory way to open the door for the characterisation of prion protein aggregates based on their "unfoldability"; the usefulness of this principle was demonstrated by Safar et al., 1998, Nature Med. 4, 1157-1165, but a bigger selection of discriminatory unfolding or solubilising substances would be highly desirable in order to achieve a thorough classification of more types of prion aggregates by this principle. Older literature suggests that scrapie is sensitive to thiocyanate, trichloroacetate and hydroxyl ions (Prusiner et al., 1981, Proc. Natl Acad. Sci., USA 78, 4606-4610). In recent years, a number of solubilising compounds have been described and have been shown to be able to dissolve or increase the protease susceptibility of prion protein aggregates ("plaques") to some extent. These compounds include antibodies, a number of small-molecule drugs (quinacrine and chlorpromazine, amphotericin B, pentosansulfate and Congo Red) lipopolyamines (Winklhofer & Tatzelt, 2000, Biol. Chem. 381, 463-469) and polyamine (cationic) dendrimers (US 6,214,366 B1, US 2002/0041859, Supattapone et al.. 1999, Proc. Natl. Acad. Sci., USA 96, 14529-14534. Supattapone et al., 2001, J. Virol. 75. 3453-3461) and, in some cases, their effect on the transmissibility or "infectivity" of treated prion proteins has also been demonstrated. It is thus known that dendrimers have some effect on their own: certain dendrimers (especially cationic dendrimers) can interact with prion aggregates and partly dissolve them, to a state where they have become protease sensible and lose infectivity (Suppatapone et al. 2001, see above). Interestingly, such dendrimers were shown to be able to both inhibit aggregate formation and to dissolve already formed aggregates to an extent where, for example, a cell culture forming prion protein aggregates were "cured" of these aggregates. It is likely that these compounds exert their effects through different mechanisms including effects on the prion protein expression in live cells, effects on the aggregate forming mechanisms (accumulation, replication and infectivity of prion proteins) and on the stability of already formed aggregates. However, it is a unique characteristic of the dendrimers that they have a clear effect on prion plaques in cell-free systems, promoting solubilisation of pre-existing aggregated prion proteins and thus affecting the aggregates directly.

Published International Patent Application, WO 00/72851, discloses a method of sterilizing objects involving contacting an object such as a medical device to be reused with a polycationic dendrimer to render conformationally altered proteins non-infectious. Furthermore, published International Patent Application, WO 01/54736, discloses an antiseptic composition for reducing infectivity of an infectious protein, preferably with pH maintained at pH 4.0 or less. The active component may be e.g. a polycationic dendrimer or a protein denaturant.

U.S patent application No. US 4,587,329 discloses dense star polymers as for example a dendrimer conjugate of a dendrimer and a carboxylic acic ester or dendrimer conjugate of formula D(R)₂.

There is still a need to develop effective agents for the treatment of protein aggregate related disease and the dendrimer compounds of Supattapone and others (see e.g. US 6,214,366) seem to be protein aggregate solubilising compounds. By contrast, the present invention combines dendrimers with protein solubilising substances thereby creating a new class of compound that have the benefit compared to the dendrimer substances of Supattapone and others that they will work at physiological pH values and that they can be tailor-made to achieve a desired level of unfolding with specific types of prion protein aggregates and optionally in combination with traditional means of solubilising proteins (chaotropes and detergents). This allows for a much wider range of unfolding/solubilising activities to be achieved, providing a much higher discriminatory power to be achieved In assays for the classification of prion protein conjugates.

Interestingly, the dendrimer conjugates of the present invention were found to work at an efficiency which was not just the addition of the protein aggregate solubilising ability of the dendrimer itself, combined with that of the protein solubilising substance by itself. Finally, the invention allows non-ionisable protein-solubilising moieties to be used in a dendrimer conjugate leading to more biological uses and less problems with toxicity than seen with the cationic dendrimers of Supattapone et al. (1999, PNAS USA 96, 14529-14534).

### DESCRIPTION OF THE INVENTION

The present invention relates to a dendrimer conjugate formed between a dendrimer and a protein solubilising substance, said protein solubilising substance having a structure which is not found in the dendrimer, and the conjugate - upon treatment of protein aggregates with the dendrimer conjugate - causing an increase by a factor of more than 1 in the solubility of the protein aggregates over that obtained upon treatment of protein aggregates under the same treatment conditions with a physical mixture of the dendrimer and protein solubilising substance, the physical mixture containing the same molar ratio of the protein solubilising substance to the dendrimer as that in the dendrimer conjugate.

The method detailed in Example 7, below, should be used to determine the solubilising effect of the dendrimer conjugates.

The present invention concerns dendrimer conjugates wherein the solubility of the protein aggregates is increased by a factor of more than 1 such as, e.g., at least 1.5 or at least 2.

### Description of Figures

Figure 1: General structures of commercially available dendrimers: PPI dendrimers (DAB dendrimers), PAMAM dendrimers and PAMAM-Starburst™ dendrimers, showing the concept of different generations and some examples of useful types of protein solubilising substances which can be coupled to the surface primary amines of the dendrimers.
Figure 2: Two examples of dendrimer conjugates both containing one free primary amino group on the surface, deriving from the attachment of the dendrimer to the solid phase during synthesis.

Top: Guanidine substituted PAMAM 3^{rd} generation dendrimer conjugate.

Bottom: Sulfonylurea substituted DAB 4^{th} generation dendrimer conjugate.

### Definitions and Abbreviations used in the text

A *dendrimer* is a molecule with a structure that extends from one or more core points through multiple generations of successive layers, with each layer having one or more branching points, to end in equivalent surface groups. They can be globular (spherical) or tree-shaped.

A *PPI dendrimer* is defined as a dendrimer consisting of poly(propyleneimine) layers built on a diaminobutane core unit. PPI dendrimers are commercially available and have well-studied physical and chemical properties.

A *PAMAM dendrimer* is defined as a dendrimer consisting of poly(amidoamine) layers built on a ethylenediamine core unit. PAMAM dendrimers are commercially available and have well-studied physical and chemical properties.

The *surface groups* of a dendrimer are those groups which appear at the end of the branches of the dendrimer. They usually occupy the outer surface of the dendrimer structure and as such, they govern the intermolecular interactions of the dendrimer (*e*.*g*. with solvents). The interaction of a dendrimer with another molecule usually occurs *via* the surface groups.

A *solid phase support* is an insoluble polymer which is functionalised to allow reagents to be bound to its surface *via* a linker entity. Solid phase chemistry simplifies the synthesis and isolation of products, and is commonly used in techniques such as HTS and combinatorial chemistry.

A *linker* is defined as a bifunctional reagent containing an anchor group to the solid phase support and an anchor group to the reagent. The anchor moieties of a linker are joined to the solid phase and substrate.

A *conjugate* is defined as an association of two or more compounds which are covalently bound together to form a new compound. Bonding occurs between the compounds so that the structure of the conjugate can be determined chemically or spectroscopically. A dendrimer conjugate is a conjugate in which one of the compounds is a dendrimer.

A *chaotrope* is a substance which destabilises the structure of a protein in solution. Chaotropes break down the hydrogen-bonded network between water molecules, allowing macromolecules more structural freedom and encouraging protein extension and denaturation.

When used in relation to the current invention, a *protein solubilising substance* is taken to mean a substance which acts upon (insoluble) protein aggregates to make them soluble in a reaction medium. Solubilising such aggregates makes them susceptible to proteases and may give beneficial effects on protein aggregate related diseases.

A *protein denaturant* is a substance which alters the secondary or tertiary structure of a protein, a process which usually destroys or reduces its activity. It is thought that they operate by disrupting non-covalent interactions within the protein.

A *protein aggregate* is an insoluble collection of proteins which have altered properties from their natural state. They often have conformation which differs from their normally soluble state. Such aggregates may be composed of nonbranching fibrillar proteins containing proteins with a β-sheet conformation. Protein aggregates are also known as "plaques".

A *protein aggregate related disease* is a disease which is linked to protein aggregates. The precise relationship between the protein aggregates and the protein aggregate related diseases is yet unclear, but they may be a symptom, a cause or another factor related to the disease. Protein aggregate related diseases are characterised in that they are all substantially incurable, and are all devastating or fatal. An important group of these diseases are neurodegenerative.

A *prion-related disease* is a protein aggregate related disease which is characterised by a build-up of insoluble prion protein in an infected animal. Prion-related diseases include new variant Creutzfeldt-Jakob disease and bovine spongiform encephalopathy.

An *amyloid-related disease* is a protein aggregate related disease which is characterised by a build-up of insoluble amyloid protein in an infected animal. Alzheimers disease is an example of an amyloid-related disease.

A *physical mixture* of two or more components is a mixture formed by combining the components in solid, liquid or solution phase. No covalent bonds are formed between the components of a physical mixture, and only weak intermolecular forces exist (e.g. H-bonds, van der Waals forces).

The terms *"reduce infectivity"* or *"disinfect"* are used here to mean reducing the ability of an entity to cause a disease. In this invention, the entities are protein aggregates and the diseases are the protein aggregate related diseases.

A *broad-spectrum protease* is a protease which acts on a wide range of proteins (*e.g*. a non-specific protease).

Within the current invention, *protease sensitivity* is used to denote the susceptibility of a protein aggregate to a protease enzyme. Normally insoluble protein aggregates will only react with a protease if first solubilised. Hence, the effectiveness of a specific substance can be assessed by treating the protein aggregate with the substance, reacting the mixture with a broad spectrum protease and then performing a blotting assay to determine the amount of protein aggregate which remains.

| | |
|---|---|
| ApoA1 | apoliprotein A1 |
| apoE | apoliprotein E |
| APP | amyloid precursor protein |
| BSE | bovine spongiform encephalopathy |
| ELISA | enzyme linked immunosorbent assay |
| HTS | High throughput screening |
| IgGL | immunoglobulin G |
| PAMAM | poly(amidoamine) |
| PEGA | polyethylene glycoldimethylacrylamide copolymer |
| PEI | poly(ethyleneimine) |
| PPI | poly(propyleneimine) |
| PrP | prion protein |
| SDS-PAGE | sodium dodecylsulfate-polyacrylamide gel electrophoresis |
| SOD | superoxide dismutase |

### Detailed description

As mentioned above, the present invention relates to a dendrimer conjugate formed between a dendrimer and a protein solubilising substance, said protein solubilising substance having a structure which is not found in the dendrimer, and the conjugate - upon treatment of protein aggregates with the dendrimer conjugate - causing an increase by a factor of more than 1 in the solubility of the protein aggregates over that obtained upon treatment of protein aggregates under the same treatment conditions with a physical mixture of the dendrimer and protein solubilising substance, the physical mixture containing the same molar ratio of the protein solubilising substance to the dendrimer as that in the dendrimer conjugate. Generally, the one or more same or different prion solubilising substances are covalently bound to the dendrimer.

The method detailed in Example 7, below, should be used to determine the solubilising effect of the dendrimer conjugates.

The prion-solubilising substance exerts a solubilising effect on prion protein aggregates as evidenced by a substantial increase in the solubility of said prion protein aggregates. The prion-solubilising efficiency of a dendrimer conjugate (EC50) is defined herein as the concentration of the dendrimer conjugate that reduces the amount of protease resistant prion protein by 50%, as compared to a control incubation without dendrimer. As seen in Example 7 this determination is conveniently performed by incubating, at specified conditions of pH, temperature, agitation and duration of incubation a prion protein aggregate containing brain homogenate with a series of concentrations of the conjugated dendrimer (including a zero concentration - non dendrimer control), followed by a standardised proteinase K treatment and analysis of the degradation product by SDS-PAGE followed by immunoblotting, quantitated by PrP-specific antibodies and densitometric scanning of the blot. The 50% efficient concentration (EC50) of the conjugated dendrimer can then be determined by inspection of the dilution curve.

The conjugated dendrimers of the present invention are characterised by having EC50 values towards susceptible prion protein aggregates in the range of 10 to 500 ug/ml, typically from 20 to 200 ug/ml, more precisely from 30-100µg/ml and optimally around 50 ug/ml under the incubation conditions specified in Example 7.

A susceptible prion protein aggregate is defined herein as the type of prion protein aggregate found in the brain of a terminally diseased or dead animal or human suffering from a prion disease and being characterised by having a very high protease stability as demonstrated by any of the assays known to anyone skilled in the art, e.g. the Prionics Western blot assay or an equivalent assay using antibodies that specifically react with the type of prion protein in question.

From this definition of a prion-solubilising substance, it is essential that the type of prion needs to be specified; in fact it is a characteristic of the conjugated dendrimers of the present invention that they have highly differential efficiencies towards different types of prion protein aggregates, and this is the background for their use as prion characterising substances as described herein.

It may also be the case that non-susceptible prion protein aggregates may be rendered susceptible by addition of certain substances, especially certain protein denaturants, including chaotropes and detergents to the incubation mixture before or concurrently with the conjugated dendrimer.

The present invention concerns dendrimer conjugates wherein the solubility of the protein aggregates is increased by a factor of more than 1 such as, e.g., at least 1.5 or at least 2.

The present invention discloses a new type of dendrimer conjugate (also denoted decorated dendrimers) that are characterised by having a high density of very efficient protein solubilising groups on their surface. The protein solubilising groups are formed by covalently binding of the dendrimer with one or more same or different protein solubilising substances.

Dendrimers of a number of well-known and well-described types at a generation number that allows substantial definition of the dendrimer are used as scaffolds upon which various protein solubilising substances are attached by facile, one- or few-step syntheses, said protein solubilising substances being selected on the basis of their ability to solubilise protein aggregates. Therefore, dendrimer conjugates according to the invention have the structure

D(R)n

wherein
D is the dendrimer
R is a radical of the protein solubilising substance which may be the same or different, and
n is an integer greater than 1.

In a particular embodiment, the R group is bound to the surface groups of the dendrimer.

### Protein solubilising substance

As mentioned above, a dendrimer conjugate according to the invention contains a protein solubilising substance. Typically, the protein solubilizing substance is a protein denaturant which may be selected from the group consisting of ureas, thioureas, sulfonylureas, semicarbazides, hydrazides, thiosemicarbazides, guanidines and chaotropes.

An interesting class of such protein solubilizing substance to be present on the dendrimer surface is derived from chaotropes and from variants thereof, including, but not limited to cationic chaotropes.

Such substances include primary amines, guanidinium groups, thiocyanates and urea groups as well as other known chaotropic groups.

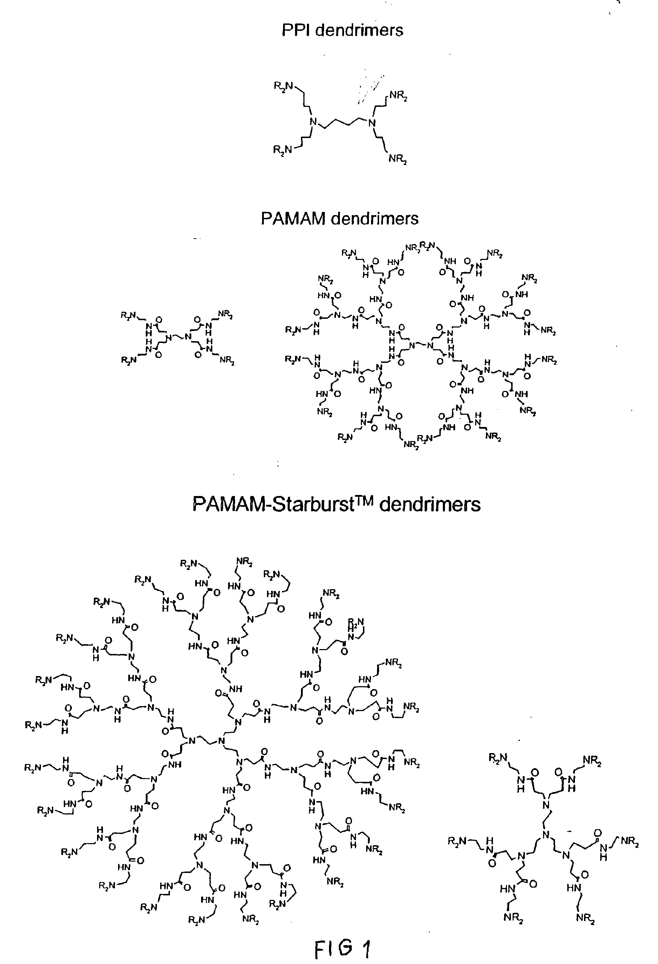

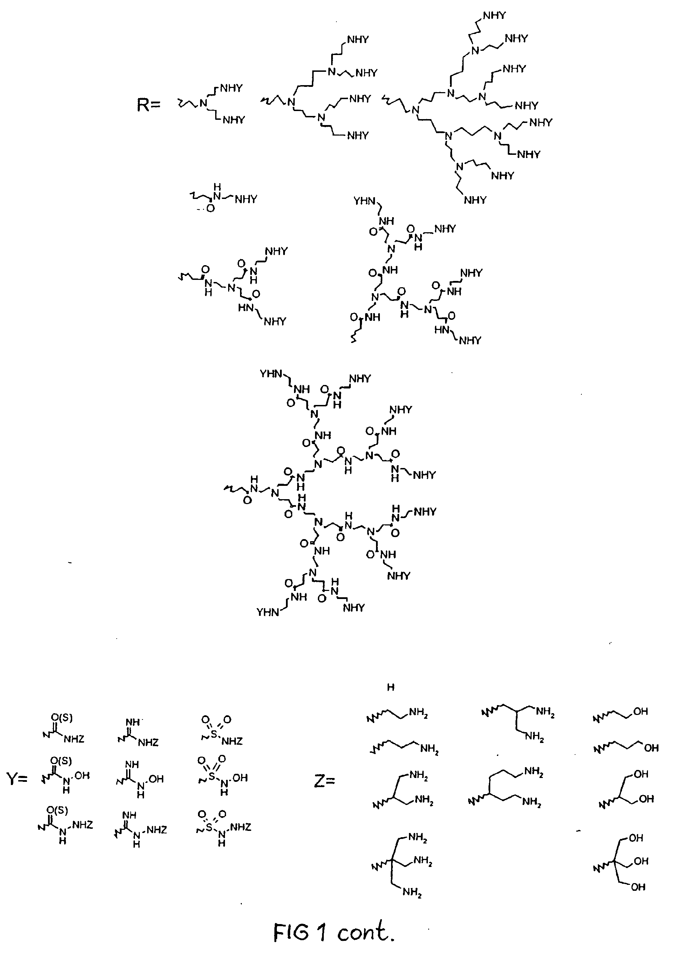

Specific protein solubilizing substances of the dendrimer conjugates of the present invention include amines, guanidinium groups, thiocyanates and urea groups as well as other known chaotropic groups as *e.g*. thiourea, sulfonylurea, hydrazide, semicarbazide and thiosemicarbazide. In one version of the invention these groups are combined as illustrated in figure 1 to provide a certain combination and a certain spatial organisation of H-bond forming and hydrophobic and hydrophilic protein solubilizing substances. This is achieved by the combination of terminal, cationic protein solubilizing substances, including guanidines and amines with other functional groups linking the whole protein solubilizing substance to the dendrimer surface.

The protein solubilizing substance is different from the repeating unit of the dendrimer to which the protein solubilizing substance is covalently bound. Otherwise the conjugate obtained is merely the next generation of dendrimer.

In specific embodiments of the invention the structure of the dendrimer conjugates are illustrated in figure 1. They provide dendrimer conjugates with a suitable combination and a spatial organisation of H-bond forming and hydrophobic and hydrophilic protein solubilising substances, achieved by the combination of terminal, cationic protein solubilising substances, including guanidines and amines ("Z" in figure 1) with another protein solubilising substance ("Y" in figure 1). In addition to furnishing the dendrimer surface with cationic protein solubilising substances adjacent to other protein solubilising substances, it is also clear that the listed "Y" groups lead to a different surface distribution of cationic protein solubilising substances than the distribution seen on a conventional cationic dendrimer.

Particular examples of protein solubilizing substances are depicted in figure 1. As can be seen these substances give the possibility of producing a wide range of different dendrimer conjugates with different densities of terminal protein solubilising substances in different arrangements and different patterns of hydrogen bonding and hydrophobic regions in the region of the substance ("Y" in figure 1). This makes possible the synthesis of dendrimer conjugates with finely tuned solubility and protein solubilising characteristics. The properties of the dendrimer conjugates are also influenced by the dendrimer type used, as PAMAM dendrimers will have a lower charge density in the interior in contrast to PPI dendrimers (polyamines) that will be more highly charged at neutral and acidic pH values.

The inclusion of such protein solubilising substances in large numbers on the dendrimers makes such dendrimer conjugates very powerful protein aggregate solubilising agents that are able to dissolve (partly or completely) protein aggregates at non-cytotoxic concentrations in a matter of few hours.

Hence, the present invention relates to dendrimer conjugate as described above, wherein R is 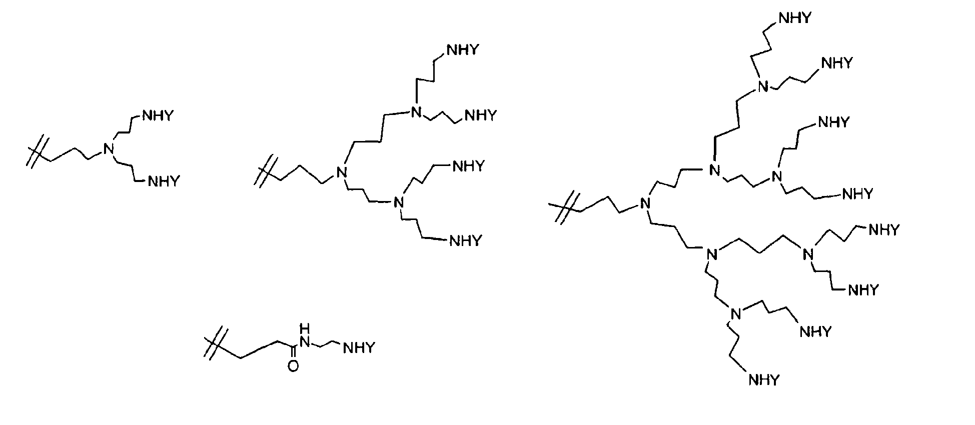 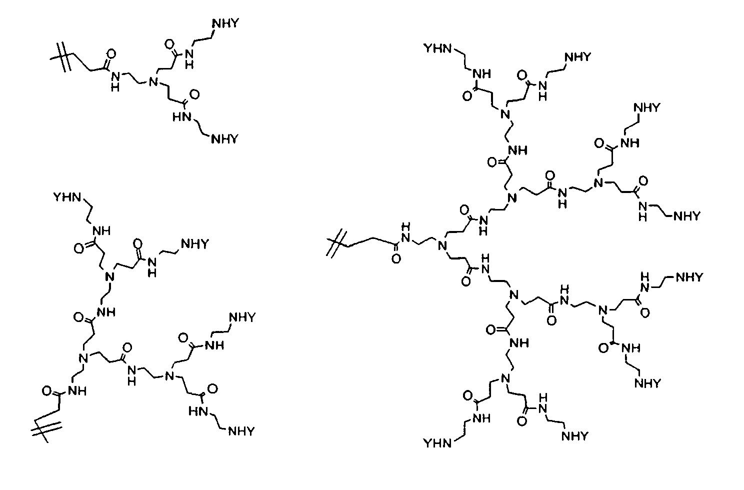 wherein Y is selected from the group consisting of 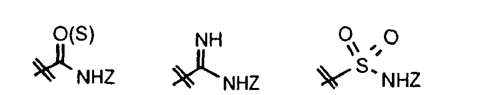 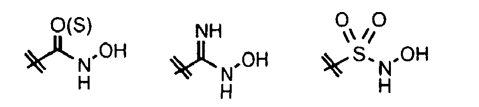 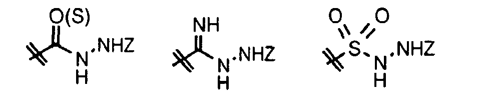 wherein Z is selected from the group consisting of

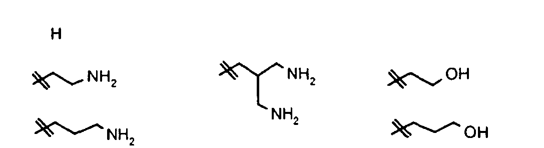

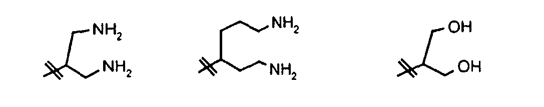

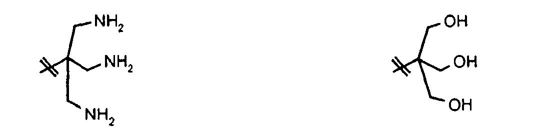

Protein solubilising substances relevant to the current invention are combinations of protein solubilising substances as depicted in figure 1 (by N- or O-substitution as appropriate), in which Z belongs to the group of ethylene amine, tris-ethylene amine and the like or hydroxyethylene, glycol or glycerol and the like.

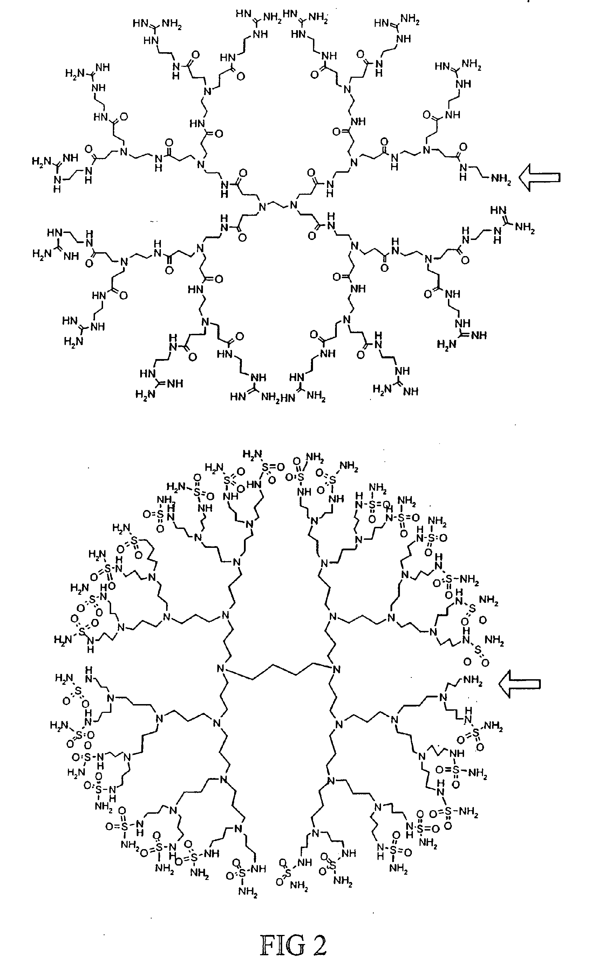

Furthermore dendrimer conjugate according the invention may contain one or more surface groups which are not occupied by a protein solubilising substance. Such surface groups are often amino groups so that, in one embodiment of the current invention, the dendrimer conjugate will contain one unmodified primary amino group on its surface (see figure 2). This amino group may be a result of the solid phase synthesis where it is bound to the linker group which is used to connect the dendrimer conjugate to the solid phase support. When the dendrimer conjugate is released from the solid phase support, the primary amino group remains.

### Dendrimer

The present invention relates to dendrimer conjugates wherein the dendrimer is a multivalent functional dendrimer having a dendritic structure that extends from one or more core points through multiple generations of successive layers, with each layer having one or more branching points, to end in surface groups. The dendrimer conjugate can be represented structurally, and thus the present invention describes dendrimer conjugates, wherein the dendrimer (D) is represented by the formula:

X-(V)ₐ-(W)_{b}

wherein X is a multifunctional segment having one or more branching points,
V is a linker or spacer group, which may be branched or linear
W is a surface group and
a and b are integers such that each linker group V terminates in one or more surface groups W.

The dendrimer conjugates of the invention can be based on different dendrimer types, which are often commercially available types like PAMAM- denrimers and PPI-(polypropyleneimine) dendrimers, and normally in the range of 2^{nd} - 4^{th} generation, for example 3^{rd} generation.

The dendrimers of the dendrimer conjugates according to the present invention may be globular or tree-shaped. In one embodiment, the generation of the dendrimer ranges from 0 to 20 such as *e.g*. from 1 to 10 or from 2 to 6. In another embodiment, the molecular mass of the unmodified dendrimers according to the present invention lies from 50 to 30000 such as *e.g*. from 100 to 20000 or from 300 to 15000. Furthermore, the dendrimer conjugates of the present invention contain dendrimers in which the number of surface groups on the dendrimer lies between 2 and 256 such as *e.g*. between 2 and 64, between 4 and 32 or between 8 and 32, such as *e.g*. 4, 8, 16, 32 or 64. Dendrimer conjugates according to the present invention are such that wherein the surface groups of the dendrimer are amine functionalities. The current invention also relates to dendrimer conjugates, wherein the dendrimer is a PPI dendrimer or a PEI dendrimer or a PAMAM dendrimer.

Additionally, the present invention describes dendrimer conjugates wherein the dendrimer has one of the following core structures 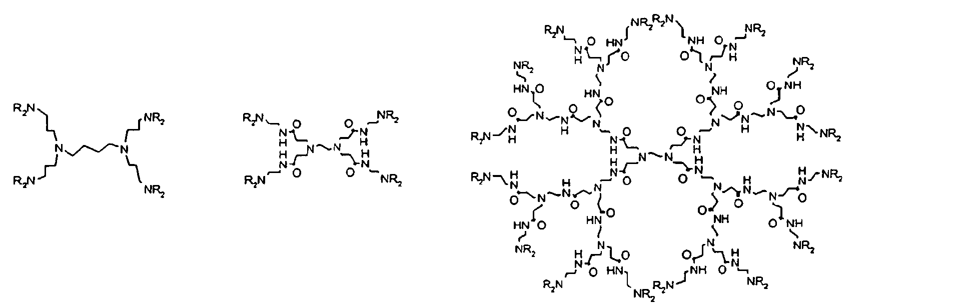 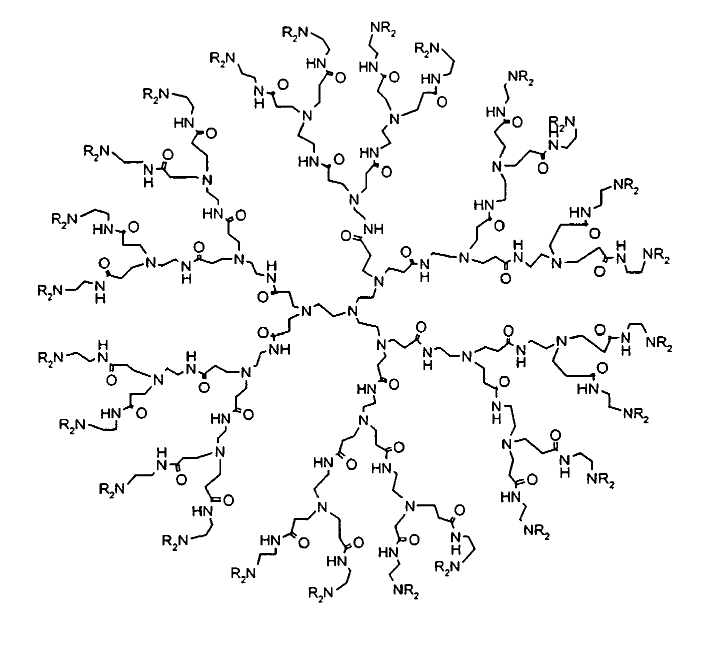 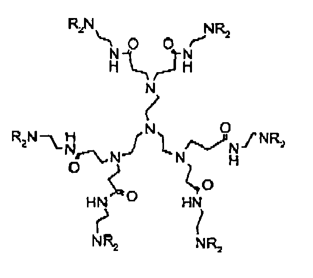 wherein R has the same meaning as previously described.

In one embodiment according to the invention, the dendrimer is a conjugate of two or more multivalent functional dendrimers as defined herein.

### Use of dendrimer conjugates

A particular use of the dendrimer conjugates of the invention is for solubilisation of protein aggregates of different protein malfolding mediated diseases, including the prion diseases. In other words, the dendrimer conjugates according to the invention are to be used for the manufacture of a medicament for the treatment of protein aggregate related diseases. Specific protein aggregate related diseases relevant to the invention are selected from the group consisting of Alzheimer's disease, Creutzfeldt-Jakob disease, variant Creutzfeldt-Jakobs disease, fatal familial insomnia, Gerstmann-Sträussler-Sheinker syndrome, PrP-cerebral amyloid angiopathy, scrapie, bovine spongiform encephalopathy, chronic wasting disease, transmissible mink encephalopathy, Pick's disease, Parkinson's disease, amyotrophic lateral sclerosis, frontotemporal dementia, diabetes type II, multiple myeloma-plasma cell dyscrasias, familial amyloidotic polyneuropathy, medullary carcinoma of thyroid, chronic renal failure, congestive heart failure, senile cardiac and systemic amyloidosis, chronic inflammation, atherosclerosis, familial amyloidosis and Huntington's disease.

Furthermore, dendrimer conjugates according to the invention are to be used in the treatment of protein aggregate related diseases, wherein the protein of the protein aggregate is selected from the group consisting of APP, Aβ peptide, α1-antichymotrypsin, tau, non-Aβ-component, presenillin 1, presenillin 2, apoE, prion protein including protease resistant prion protein, SOD, Pick body, α-synuclein, anylin, IgGL-chain, transthyretin, procalcitonin, β2-microglobulin, atrial natriuretic factor, serum amyloid A, ApoA1, Gelsolin and Huntington. In a particular embodiment, the protein aggregate related disease is a prion-related disease. In a further embodiment, the protein aggregate related disease is an amyloid-related disease.

By the current invention a method is provided for treating, preventing and/or diagnosing a protein aggregate related disease in a subject, the method comprising administering to the subject in need thereof a sufficient amount of a dendrimer conjugate. Also disclosed is the use of dendrimer conjugates according to the invention in the preparation of a medicament for use in the treatment, prophylaxis and/or diagnosis of protein aggregate related diseases.

Another use of the dendrimer conjugates of the invention is for treatment of cells or animals, including humans to prevent the build-up of protein aggregates and to promote the clearance of already formed protein aggregates. Therefore, the present invention enables a method of preventing the formation of protein aggregates in cells or animals, the method comprising the treatment of cells or animals with a dendrimer conjugate according to the present invention. A particular use of dendrimer conjugates of the present invention is the preparation of a medicament for the treatment or prophylaxis of prion diseases. A further use of dendrimer conjugates according to the invention is to reduce the infectivity of prion proteins. A typical example of this is the solubilisation of scrapie prion protein aggregates in a homogenate from a sheep brain sample from a scrapie-affected sheep.

It is envisaged that different strains of prions (or other kinds of protein aggregates) will have different susceptibilities towards the solubilising effect of the dendrimer conjugate. In particular, sheep prions derived from bovine spongiform encephalopathy plaques (upon exposure of sheep to the BSE agent) will be discernible from scrapie prion protein aggregates in their susceptibility to solubilisation by dendrimer conjugate. To detect these differences in susceptibility to solubilisation a method is disclosed which comprises incubating the dendrimer conjugate-treated prion protein aggregates with a broad spectrum protease, as *e.g*. proteinase K for a suitable time and then detecting remaining prion protein by SDS-PAGE and immunoblotting with prion-specific antibodies e.g. by the Prionics assay as known to a person skilled in the art. Susceptible prions strains will then disappear faster than less susceptible prion strains.

Hence, the dendrimer conjugates according to the invention are to be used for identifying prion protein aggregates. Furthermore, the dendrimer conjugates of the invention are to be used for classifying the protein aggregates into specific strains according to their susceptibility to the treatment described below. The invention also enables a method of identifying and/or classifying protein aggregates in a mammal, the method comprising the steps of:
a) treating the protein aggregates with a dendrimer conjugate
b) analysing one or more products of step a)

In one embodiment of the above method, step b) comprises the steps of:
I. incubating the treated protein aggregates from step a) with a broad spectrum protease such as *e.g*. proteinase K
II. detecting remaining protein aggregates by one or more methods selected from the group comprising: SDS-PAGE and immunoblotting with protein-specific antibodies, ELISA, immunoelectrophoresis and immunohistochemistry.

In another embodiment the method comprises using conformationally sensitive antibodies that will only react with unfolded protein. In this type of assay susceptible strains will give rise to a signal while less susceptible strains will give rise to less signal. In other words, in a second embodiment of the above method, step b) of the method comprises incubating the treated protein aggregates from step a) with an antibody which is sensitive to changes in the structure of a protein present in the protein aggregate. In a further embodiment, the dendrimer conjugate itself is labelled, for example with a suitable conformationally sensitive fluorophore enabling the sensitive detection of changes in dendrimer spatial structure, which is believed to occur in a strain specific way upon contacting the dendrimer with prion protein aggregates of different strains or types.

Additionally, the method of identifying and/or classifying protein aggregates may additionally comprise the step of further treating the treated protein aggregates from step a) with a protein denaturant such as *e.g*. urea between steps a) and b). This serves to further solubilise an aggregate before the protease treatment and such an additional step discriminates more effectively between protein aggregates from two different sources.

The method of identifying and/or classifying protein aggregates may further comprise the steps of
i) repeating steps a) and b) with a different dendrimer conjugate, and
ii) optionally comparing results from the dendrimer conjugates to obtain information on the origin of the protein aggregates.

Normally step ii) is comprised in the method. Specifically, the protein aggregate to be identified or classified may be a prion protein aggregate.

An alternative use of the dendrimer conjugates of the present invention is for the decontamination of surfaces, medicines, food, devices, tools and feed-stuff by treatment with dendrimer conjugates to remove or reduce substantially prion infectivity. Hence, a method is disclosed for disinfecting an object, the method comprising contacting the object with a composition containing a dendrimer conjugate according to the invention. Additionally, a method for removing protein aggregates from food that originates from an animal is related, the method comprising contacting the food with a composition containing a dendrimer conjugate. The dendrimer conjugates of the invention may be used in the disinfection of material which has been contaminated with protein aggregates, such as e.g. prion protein aggregates.

### Synthesis of Dendrimer Conjugates

Methods for the synthesis of dendrimer conjugates are also included in this invention. As a general method for synthesising dendrimer conjugates, the dendrimer is first coupled to a suitable solid phase including polystyrene-based resins or PEGA resins through a suitable, selectively cleavable linker moiety. In other words, the invention also relates to a method for the preparation of a dendrimer conjugate, wherein the preparation is carried out while the dendrimer is grafted to a solid phase support through a linker entity. In a particular embodiment of the preparation method according to the current invention, the linker entity is an acid labile linker, such as *e.g*. chlorotritylchloride, Wang, Rink, Sieber or related linkers. In a further embodiment, solid phase support is selected from the group comprising polystyrene, modified polystyrene and PEGA. Once bound to the solid phase, the dendrimer is then treated with a precursor of the desired protein solubilising group. The final dendrimer conjugate is cleaved off the solid phase, and eventual protective groups are cleaved off either during cleavage from solid phase or subsequently. This method of synthesis is amenable to combinatorial chemistry and library screening (HTS) for activity against protein aggregates.

The dendrimer conjugates of the present invention are substantially soluble in water or aqueous buffers and interesting dendrimer conjugates carry a positive net charge at neutral pH. Furthermore, by presenting a high number of protein solubilising groups (chaotropes or other types) on their surfaces, said dendrimers achieve a substantially increased protein aggregate solubilising effect compared to the same solubilising substance employed as a solubliser on its own. It is furthermore possible by specific combinations of the Y and Z groups of the surface groups to fine-tune the dendrimer conjugates of the invention to exhibit a desired degree and specificity of protein aggregate solubilising ability.

When the dendrimer conjugate is decorated with multiple sulfonylurea (sulfamides) groups, it may be synthesised by reacting the dendrimer (often in solution) with sulfonylamide (>SO₂NHR) reagents e.g. chlorosulfonyl-isocyanate, halo-sulfamides, chlorsulfonyl-*tert*butylsulfamate or other sulfonylamide reagent known for a person skilled in the art. Protection groups present at the dendrimer surface can subsequently be removed by e.g. acidolysis or other condiditons known to a person skilled in the art.

When the dendrimer conjugate is decorated with multiple guanidine end groups, it may be synthesised by reacting the dendrimer with e.g Di-boc-S-methyl-isothiourea,.Di-Boc-thiourea and condensing agents such as carbodiimides, phospohonium salts or other condensing reagents well known to a person skilled in the art. Subsequently, the Boc-protection can be removed by acidic treatment e.g. with trifluoroacetic acid in dichloromethane.

When the dendrimer conjugate is decorated with multiple thiourea end groups it may be synthesised by reacting the dendrimer (often in solution) with thiocarbamoyl (-(C=S)NHR) reagents e.g. alkyl-thiocarbamoyl halides or other thiocarbamoyl reagent known to a person skilled in the art. Protection groups present at the dendrimer surface can subsequently be removed by e.g. acidolysis or other condiditons known to a person skilled in the art.

When the dendrimer conjugate is decorated with multiple urea end groups, it may be synthesised by reacting the dendrimer (often in solution) with carbamoyl (-(C=O)NHR) reagents e.g. alkyl-carbamoyl halides or other carbamoyl reagents known to a person skilled in the art. Protection groups present at the dendrimer surface can subsequently be removed by e.g. acidolysis or other conditions known to a person skilled in the art.

Further embodiments of the present invention can be seen in the following Examples.

### EXAMPLES

The following examples provide evidence of the feasibility of the invention but are not meant to limit the invention to the uses and the embodiments presented in the examples.

### General: Synthesis of isothiocyanates.

Carbondisulfide (10 equiv) is dissolved in DCM and 1.1 equiv peptide coupling reagent (e.g. PyBOP or TFFH) is added followed by 1 equiv protected amine and 3 equiv NMM. The mixture is stirred for 30 min at r.t. DCM and carbondisulfide is evaporated in vacuo and the crude isothiocyanate is ready for further synthesis.

### EXAMPLE 1: Solid phase synthesis of thiourea-dendrimer conjugates.

Amino terminated dendrimer (1.5 equiv) is added to a chlorotrityl-chloride resin (1 equiv) in DCM. The suspension is shaken for 2h at r.t. Residual chlorotrityl groups are capped with a DCM/methanol/NMM 17:2:1 mixture. The resin is washed with DCM (5 times) and NMP (5 times). TNBSA test shows positive. The amino terminated dendrimer bound to the chlorotrityl-chloride resin is suspended in NMP and an adequately protected isothiocyanate (5 equiv relative to numbers of surface amines on the dendrimer) and the mixture is shaken for 2 days at r.t. The resin is washed with NMP (10 times) and DCM (5 times). TNBSA test shows negative. The dendrimer conjugate is deprotected and cleaved off the resin with 50 % TFA in DCM for 2 h at r.t. The dendrimer conjugate is triturated with diethyl ether.

### EXAMPLE 2: Solid phase synthesis of guanidine-dendrimer conjugates.

The amino-terminated dendrimer bound to a chlorotrityl-chloride resin is prepared as in Example 1 and suspended in NMP and N-Boc-protected S-methyl-isothiourea (5 equiv relative to number of surface amines on the dendrimer) is added. The suspension is shaken for 16h at 50°C. The resin is washed with NMP (10 times) and DCM (5 times). TNBSA test shows negative. The dendrimer conjugate is deprotected and cleaved off the resin with 50% TFA/DCM. The dendrimer conjugate is triturated with diethyl ether.

### EXAMPLE 3: Solid phase synthesis of sulfonylurea-dendrimer conjugates.

Amino terminated dendrimer bound to a chlorotrityl-chloride resin is prepared as in Example 1 and resuspended in dry DCM/pyridine 1:1 mixture and sulfurylchloride (5 equivalents relative to number of surface amines on the dendrimer). The mixture is shaken for 3h at r.t. The resin is washed with dry DCM (3 times), and a suitably protected amine (5 equiv relative to number of surface amines) is added and the suspension is shaken overnight at r.t. The dendrimer conjugate is cleaved off the resin together with protecting groups at the dendrimer with 50% TFA in DCM for 2h at r.t. The dendrimer conjugate is triturated with diethyl ether.

### EXAMPLE 4: Synthesis of dendrimer conjugates with guanidine end groups:

Dendrimer (endgroup concentration 0.26 mmol) was dissolved in DMF (0.400 ml). N, N'-DiBoc-S-methylisothiourea 2 (0.15 g, 0.51 mmol) was added, followed by diisopropylethylamine (DIPEA) (0.1 ml, 0.56 mmol). The suspension was shaken for 2 days at 40 °C, filtered and the supernatant poured into MilliQ water (2ml) causing precipitation. After centrifugation, the supernatant is removed and the residual product is suspended in 95% aqueous TFA (1 ml) and shaken 16h at r.t. The mixture was added dropwise to stirred diethyl ether (5 ml) causing precipitation. The supernatant is removed and the residue was lyophilised from water 3 times to remove residual TFA. Yield. (n=8): 0.047 g; (n=16): 0.060g; MS (MALDI-TOF)(n=8): found: 1108.5

### EXAMPLE 5: Dendrimers with sulfonylurea end groups:

1. Chlorosulfonyl isocyanate (CSI, 3.5 mmol, 0.30mL) was dissolved in dry dichloromethane (DCM, 20 mL). The mixture was cooled to 0 °C on an ice-bath. *Tert-*butanol (3.5 mmol, 0.26g) in DCM (2mL) was added dropwise, and the mixture was stirred 20 min at this temperature. Dendrimer (end group concentration 3.12 mmol) in DCM (2mL) was added dropwise at this temperature and the mixture was stirred overnight, slowly warming up to room temperature. The solvent was removed by evaporation and the residue was taken up in ethyl acetate (20 mL) and washed with 1 M NaOH and brine The organic layer was dried (Na₂SO₄) and evaporated in vacuo. The Boc-groups at the product was removed by 50% TFA in DCM overnight, followed by repeated evaporation from diethyl ether. The final deprotected product was characterised by ¹H-NMR and MALDI-TOF MS.
2. Chlorosulfonyl isocyanate (CSI, 3.5 mmol, 0.30mL) was dissolved in dry dichlrormethane (DCM, 20 mL). The mixture was cooled to 0 °C on an ice-bath. *Tert-*butanol (3.5 mmol, 0.26g) in DCM (2mL) was added dropwise, and the mixture was stirred 20 min at this temperature. The mixture was cooled to -10 °C on an ice-ethanol bath and ethyl-4-aminobutyrate-hydrochloride (3.9 mmol) was added followed by dropwise addition of DIPEA (3.9 mmol) at this temperature and the mixture was stirred overnight slowly warming up to room temperature. The solvent was removed by evaporation and the residue was taken up in ethyl acetate (20 ML) and washed with 1 M NaOH and brine The organic layer was dried (Na₂SO₄) and evaporated *in vacuo.* The ethyl ester was cleaved by hydrolysis using LiOH, and the product characterised by ¹H- and ¹³C-NMR and MALDI-TOF MS.
3. Chlorosulfonyl isocyanate (CSI, 3.5 mmol, 0.30mL) was dissolved in dry dichloromethane (DCM, 20 mL). The mixture was cooled to 0 °C on an ice-bath. *Tert-*butanol (3.5 mmol, 0.26g) in DCM (2mL) was added dropwise, and the mixture was stirred 20 min at this temperature. The mixture was cooled to -10°C on an ice-ethanol bath and glycine-*tert*-butylester-hydrochloride (3.9 mmol) was added followed by dropwise addition of DIPEA (3.9 mmol) at this temperature and the mixture was stirred overnight slowly warming up to room temperature. The solvent was removed by evaporation and the residue was taken up in ethyl acetate (20 mL) and washed with 1 M NaOH and brine The organic layer was dried (Na₂SO₄) and evaporated in vacuo. The *tert*butylester and boc-group were cleaved by 50% TFA in DCM overnight, followed by repeated evaporation from diethyl ether and the product was characterised by 1H- and 13C-NMR and MALDI-TOF MS.
4. The carboxylic acid derivatives (2) and (3) containing sulfonylurea were subsequently coupled to the surface aminogroups on the dendrimer by a peptide coupling reagent and DIPEA, in case of compound (2), the boc-groups was subsequently cleaved by TFA in DCM overnight. Sulfonylurea decorated dendrimers containing an alkyl spacer between dendrimer and hydrogen bonding end group were obtained as products and characterised by ¹H-NMR and MALDI-TOF MS.

### EXAMPLE 6: Dendrimers with thiourea and urea end groups:

1. Ethyl-4-aminobutyrate-hydrochloride (3.9 mmol) was dissolved in DMF; carbon disulfide (39 mmol) was added followed by HBTU (3.9 mmol) and DIPEA (16 mmol). The mixture was stirred 30 min at r.t. and the mixture was poured into water and extracted with DCM. The organic layer was washed with water, 1 M KHSO₄ and brine. After drying (Na₂SO₄) the solvent was removed *in vacuo,* and the residual isothiocyanate was taken up in aqueous ammonia for 2 hours. The mixture was extracted with ethyl acetate; the organic layer was dried (Na₂SO₄) and evaporated *in vacuo.* The residual ethyl ester was cleaved by LiOH, followed by workup and products characterised by ¹H-NMR and MS.
2. Ethyl-4-aminobutyrate-hydrochloride (3.9 mmol) was dissolved in DCM, carbonyldiimidazole (3.9 mmol) or di-*tert*butyl-tricarbonate (3.9 mmol) together with DIPEA (8 mmol) was added and the miuxture stirred for 30 min, followed by evaporation of the solvent. The residual isocyanate was taken up in aqueous ammonia for 2 hours. The mixture was extracted with ethyl acetate, the organic layer was dried (Na₂SO₄) and evaporated in vacuo. The residual ethyl ester was cleaved by LiOH, followed by workup and products characterised by ¹H-NMR and MS.
3. The carboxylic acid derivatives (1) and (2) containing thiourea or urea were subsequently coupled to the surface amino groups on the dendrimer by a peptide coupling reagent and DIPEA in DCM overnight. Thiourea and urea decorated dendrimers containing an alkyl spacer between dendrimer and the hydrogen bonding endgroup were obtained as products and were characterised by ¹H-NMR and MS.

### EXAMPLE 7: Increase in the solubility of hamster prion protein aggregates after treatment with dendrimer conjugates.

Hamsters are inoculated intracereberally with the 263K hamster-adapted scrapie strain and monitored closely until onset of clear clinical signs. Then the animals are killed and the brain is retrieved and a 10 (w/v) % suspension is homogenised in water by a 1 minute treatment (20.000 rpm) with an Omni Homogenizer (Omni International Inc., VA, USA). The resulting brain homogenate is subjected to proteinase K-treatment followed by immunoblotting developed by the 3F4 monoclonal antibody and peroxidase-conjugated anti mouse secondary antibodies visualised by a chemiluminescent substrate.

To test the applicability of dendrimer conjugates for solubilising prion aggregates towards proteinase K, the brain homogenate is first incubated with the dendrimer conjugate in question in a range of concentrations. Incubation can be performed at different pH values, including acidic pH values and in the presence or absence of mild detergents like NP40 and at room temperature or at 37 °C.

Control incubations include:
- no addition of dendrimer or dendrimer conjugate or protein solubilising substance,
- addition of the unconjugated dendrimer,
- addition of the protein solubilising substance corresponding to the protein solubilising substance on the dendrimer
- addition of said protein solubilising substance plus unconjugated dendrimer

Following this incubation, samples are neutralised if necessary and Sarkosyl is added, for example to a final concentration of 2%.

Hereafter the sample is subjected to proteinase K (20 ug/ml, total protein/enzyme ratio typically 25/1) for 1 hour at 37°C, stopping the protease treatment by Pefabloc or by PMSF in ethanol. Then samples are immunoblotted as above. Some samples are subjected to treatment with traditional denaturants as e.g. urea before treatment with proteinase K.

In a typical experiment, homogenates are adjusted to 1 mg/ml and to 1 % Triton X-100, 50 mM Tris acetate pH 7.5, and dendrimer conjugates are added in a range of concentrations (typically 10, 20, 50, 100 and 200 µg/ml) from a 3 mg/ml stock solution in water. Incubation is performed for 3 hours at 37 °C with agitation, whereafter 1 volume of 0.3 M NaCl and 4% Sarkosyl and Proteinase K to 20 µg/ml is added and incubated for 1 additional hour at 37 °C. This incubation is then stopped by Pefabloc (5 µM) and the samples subjected to Western Blotting.

Results show that brain homogenates containing susceptible prion protein aggregates and treated by dendrimer conjugates are dramatically more susceptible to proteinase K-degradation than non-treated homogenates as seen by the disappearance of 3F4-reactive bands after proteinase K-treatment and immunoblotting. The prion protein solubilising efficiency of the conjugated dendrimer in question is determined from a plot of the total counts of the densitometric scanning of the blot against the conjugated dendrimer concentration used for treating the homogenate before the proteinase K digestion step. Here the 50% efficient concentration (EC50) is defined as the concentration of dendrimer necessary to obtain removal of 50% of the prion protein compared to the control not pretreated with the conjugated dendrimer.

A typical EC50 for the conjugated dendrimers of the present invention towards susceptible prion protein aggregates is 50 µg/ml or below.

### EXAMPLE 8: Decreased infectivity of dendrimer conjugate-treated aggregated prion protein compared to untreated prion protein aggregates.

Brain homogenates obtained as in the previous example are inoculated intracerebrally into hamsters and the development of clinical disease is followed. It will be seen that brain homogenates first treated by dendrimer conjugates are considerably less efficient in transferring disease than non-treated dendrimers. As a control a group of hamsters is inoculated intracerebrally with the dendrimer conjugates used for treating the brain homogenates and in concentrations similar to those found in the brain homogenates, in order to demonstrate lack of toxicity *in vivo* It is expected that the dendrimer conjugates of the invention do not show a substantial *in vivo* toxicity.

### EXAMPLE 9: Curing of PrP^{Sc} - "infected" cells of protease resistant prion protein aggregates by treatment of cell cultures with dendrimer conjugates.

The persistently scrapie-infected mouse neuroblastoma-derived cell line SMB is used. Cells are maintained in culture and exposed to various types of dendrimer conjugates at different concentrations and for different time periods to assess the cytotoxicity of the compounds. Cytotoxicity is assessed by measurement of formazan dye reduction (MTS) and by observation of cell morphology. It is expected that dendrimer conjugates at non-cytotoxic concentrations and after relatively short exposure times, as for example 5 hours or even 2 hours will remove protease-resistant prion protein aggregates from the culture as seen by the disappearance of protease-resistant anti-prion protein reactive material after SDS-PAGE and immunoblotting as above.

### EXAMPLE 10: Halting a scrapie prion infection in hamsters by treating PrP^{Sc} inoculated hamsters with dendrimer conjugates at various time points after inoculation.

Hamsters are inoculated intracerebrally with 263 K as above. At different times after inoculation as well as just before inoculation, different groups of animals are injected intracerebroventrically with different amounts of different types of dendrimer conjugates. It is expected that an optimal dendrimer conjugate treatment protocol can be identified in which the development of protease resistant prion protein in inoculated hamsters is inhibited totally or substantially.

### EXAMPLE 11: Differentiating between two types of prion protein aggregates by their different susceptibility towards dendrimer conjugates.

Hamsters are inoculated intracerebrally with 263 K and brain homogenates are prepared as above. BSE prion protein aggregates are obtained from brain samples from naturally BSE infected cattle (available at the Danish Veterinary Institute).

Both types of samples are subjected to a range of treatment protocols by dendrimer conjugates and subjected to proteinase treatment and SDS-PAGE immunoblotting as above. Some treatment protocols combine treatment with dendrimer conjugate with a subsequent solubilising step for example with urea at at high concentration, for example 9 M or 8 M, before the proteinase treatment.

The same kind of experiment will be performed with brain homogenates from sheep with scrapie and from sheep with experimentally induced BSE.

It is expected that an optimal group of treatment protocols will lead to immunoblots showing substantial differences in the proteinase susceptibility of 263K as compared to BSE prions, as seen by a clear difference in the intensity of bands on the immunoblot between the two prion types. It is also expected that, by an optimised protocol as explained above, BSE-induced prions in sheep can be differentiated from scrapie-derived prions in sheep brain.

## Claims

1. A dendrimer conjugate formed between a dendrimer and a protein solubilising substance, having the structure
D(R)n
wherein
D is the dendrimer;
R is a radical of the protein solubilising substance which may be the same or different, and is selected from 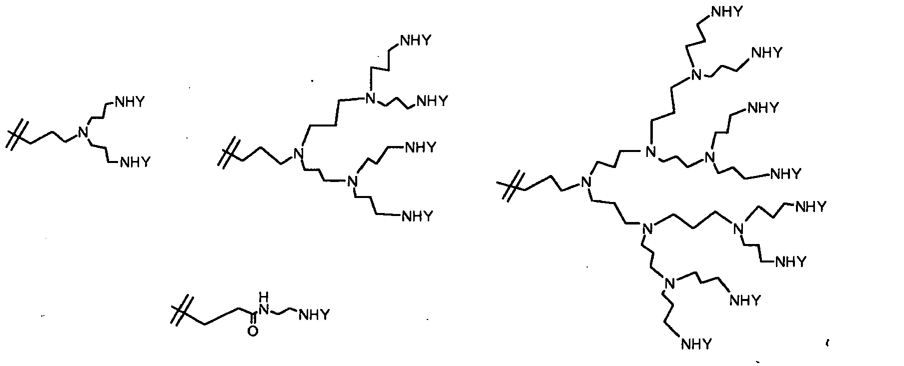 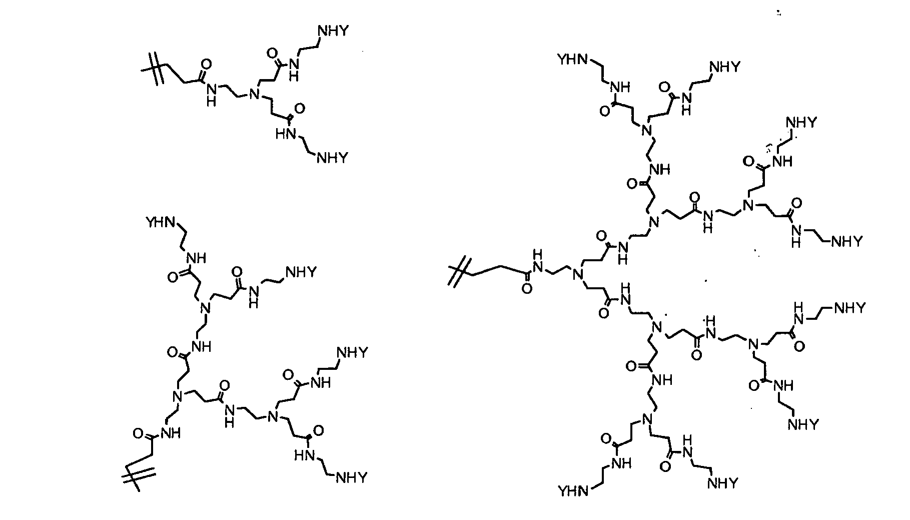 wherein Y is selected from the group consisting of 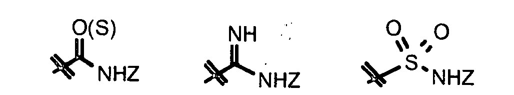 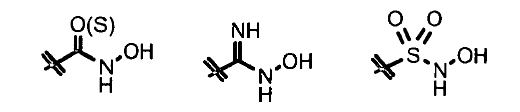 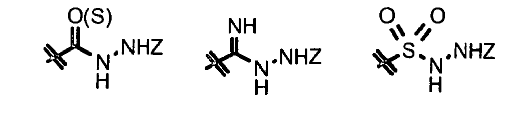 wherein Z is selected from the group consisting of 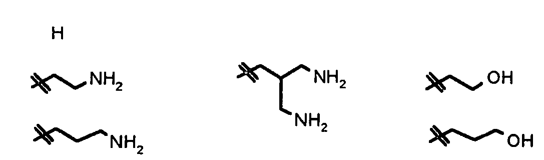 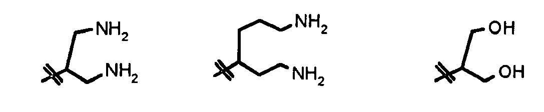 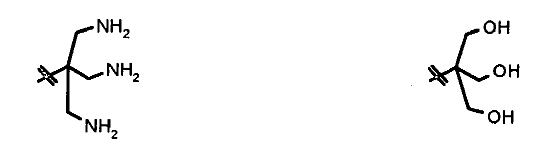 and, n is an integer greater than 1;
said protein solubilising substance having a structure which is not found in the dendrimer, and the conjugate - upon treatment of protein aggregates with the dendrimer conjugate - causing an increase by a factor of more than 1 in the solubility of protein aggregates over that obtained upon treatment of protein aggregates under the same treatment conditions with a physical mixture of the dendrimer and protein solubilising substance, the physical mixture containing the same molar ratio of the protein solubilising substance to the dendrimer as that in the dendrimer conjugate.

2. A dendrimer conjugate according to claim 1, wherein the dendrimer is covalently bound to one or more same or different protein solubilising substances.

3. A dendrimer conjugate according to any of claims 1-2, wherein the protein solubilising substance is a protein denaturant.

4. A dendrimer conjugate according to claim 3, wherein the protein denaturant is selected from the group consisting of ureas, thioureas, sulfonylureas, semicarbazides, hydrazides, thiosemicarbazides, guanidines and chaotropes.

5. A dendrimer conjugate according to any of claims 1-4, wherein the solubility of the protein aggregates is increased by a factor of at least 1.5 or at least 2.

6. A dendrimer conjugate according to any of claims 1-5 containing one or more surface groups which are not occupied by a protein solubilising substance

7. A dendrimer conjugate according to any of claims 1-6, wherein the dendrimer (D) is a multivalent functional dendrimer having a dendritic structure that extends from one or more core points through multiple generations of successive layers, with each layer having one or more branching points, to end in surface groups.

8. A dendrimer conjugate according to claim 7, wherein the dendrimer (D) is represented by the formula:
X-(V)ₐ-(W)_{b}
wherein X is a multifunctional segment having one or more branching points,
V is a linker or spacer group, which may be branched or linear
W is a surface group and
a and b are integers such that each linker group V terminates in one or more surface groups W.

9. A dendrimer conjugate according to any of claims 7 or 8, wherein the dendrimer is globular or tree-shaped.

10. A dendrimer conjugate according to any of claims 7-9, wherein the generation of the dendrimer ranges from 0 to 20.

11. A dendrimer conjugate according to any of claims 7-10, wherein the molecular mass of the unmodified dendrimer lies from 50 to 30000.

12. A dendrimer conjugate according to any of claims 7-11, wherein the number of surface groups on the dendrimer lies between 2 and 256.

13. A dendrimer conjugate according to any of claims 7-12, wherein the surface groups of the dendrimer (D) are amine functionalities.

14. A dendrimer conjugate according to any of claims 7-13, wherein the dendrimer is a PPI dendrimer or a PEI dendrimer or a PAMAM dendrimer.

15. A dendrimer conjugate according to any of claims 7-14, wherein the dendrimer has one of the following core structures 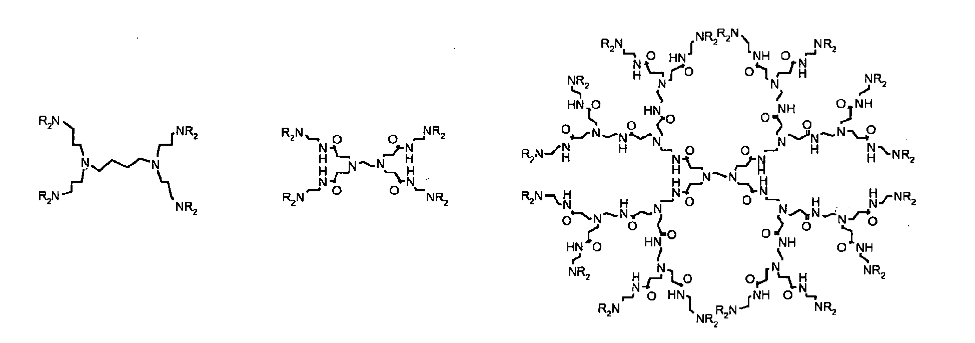 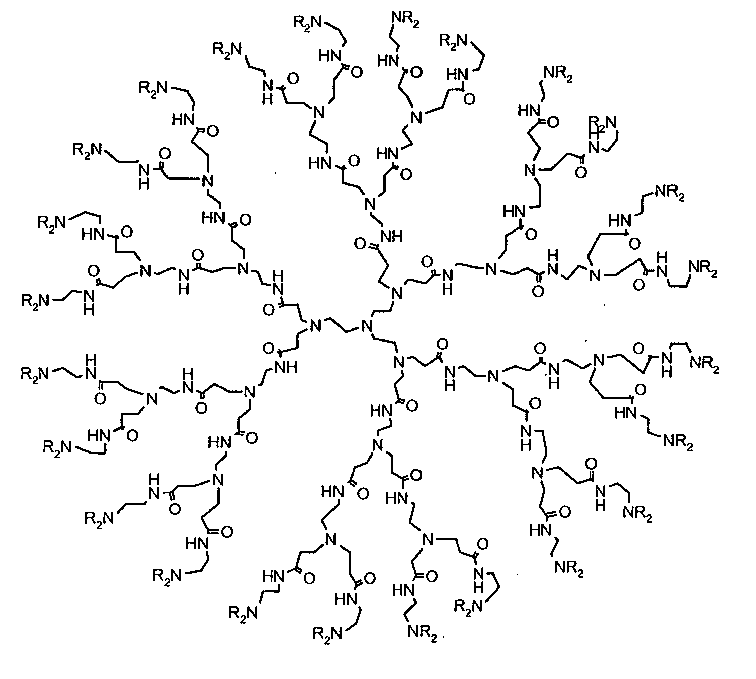 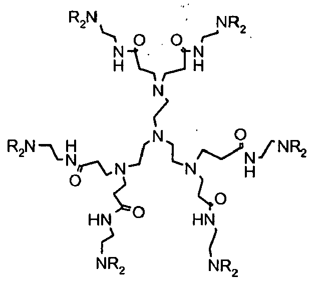 wherein R has the same meaning as in claim 1.

16. A dendrimer conjugate according to any of claims 1-6, wherein the dendrimer (D) is a conjugate of two or more multivalent functional dendrimers as defined in claims 7-15.

17. A dendrimer conjugate according to any of the preceding claims, which has an EC50 value of 10-500µg/ml.

18. Use of a dendrimer conjugate according to any of claims 1-17 for the manufacture of a medicament for the treatment of protein aggregate related diseases.

19. Use according to claim 18, wherein the protein aggregate related disease is selected from the group consisting of Alzheimer's disease, Creutzfeldt-Jakob disease, variant Creutzfeldt-Jakobs disease, fatal familial insomnia, Gerstmann-Sträussler-Sheinker syndrome, PrP-cerebral amyloid angiopathy, scrapie, bovine spongiform encephalopathy, chronic wasting disease, transmissible mink encephalopathy, Pick's disease, Parkinson's disease, amyotrophic lateral sclerosis, frontotemporal dementia, diabetes type II, multiple myeloma-plasma cell dyscrasias, familial amyloidotic polyneuropathy, medullary carcinoma of thyroid, chronic renal failure, congestive heart failure, senile cardiac and systemic amyloidosis, chronic inflammation, atherosclerosis, familial amyloidosis and Huntington's disease.

20. Use according to any of claims 18 or 19, wherein the protein of the protein aggregate is selected from the group consisting of APP, Aβ peptide, α1-antichymotrypsin, tau, non-Aβ-component, presenillin 1, presenillin 2, apoE, prion protein including protease resistant prion protein, SOD, Pick body, α-synuclein, anylin, IgGL-chain, transthyretin, procalcitonin, β2-microglobulin, atrial natriuretic factor, serum
amyloid A, ApoA1, Gelsolin and Huntington.

21. Use according to any of claims 18 or 19, wherein the protein aggregate related disease is a prion-related disease.

22. Use according to any of claims 18 or 19, wherein the protein aggregate related disease is an amyloid-related disease.

23. Use of dendrimer conjugates according to any of claims 1-17 to reduce the infectivity of prion proteins in vitro.

24. Use of dendrimer conjugates according to any of claims 1-17 in disinfection of material which has been contaminated with protein aggregates.

25. Use according to claim 24, wherein the protein
aggregates are prion protein aggregates.

26. A method of identifying and/or classifying protein aggregates in a mammal, the method comprising the steps of:
a) treating the protein aggregates with a dendrimer conjugate as defined in claims 1-17
b) analysing one or more products of step a)

27. A method according to claim 26 wherein step b) comprises the steps of
I. incubating the treated protein aggregates from step a) with a broad spectrum protease such as *e.g*. proteinase K
II. detecting remaining protein aggregates by one or more methods selected from the group comprising: SDS-PAGE and immunoblotting with protein-specific antibodies, ELISA, immunoelectrophoresis and immunohistochemistry.

28. A method according to claim 26 wherein step b) comprises incubating the treated protein aggregates from step a) with an antibody which is sensitive to changes in the structure of a protein present in the protein aggregate.

29. A method according to claim 26 additionally comprising the step of further treating the treated protein aggregates from step a) with a protein denaturant such as *e.g*. urea between steps a) and b).

30. A method according to claim 26 further comprising the steps of
i) repeating steps a) and b) with a different dendrimer conjugate, and
ii) optionally comparing results from the dendrimer conjugates to obtain information on the origin of the protein aggregates.

31. Use of dendrimer conjugates according to any of claims 1-17 for identifying prion protein aggregates.

32. Use of dendrimer conjugates according to any of claims 1-17 for classifying the protein aggregates into specific strains according to their susceptibility to the method described in claim 26.

33. Use according to claim 32, wherein the protein
aggregates are prion protein aggregates.

34. A method for disinfecting an object, the method comprising contacting the object with a composition containing a dendrimer conjugate according to any of claims 1-17.

35. A method for removing protein aggregates from food that originates from an animal, the method comprising contacting the food with a composition containing a dendrimer conjugate according to any of claims 1-17.

36. Use of dendrimer conjugates according to any of claims 1-17 in the preparation of a medicament for use in the prophylaxis and/or diagnosis of protein aggregate related diseases.

37. A method for the preparation of a dendrimer conjugate according to any of claims 1-17 wherein the preparation is carried out while the dendrimer (D) is grafted to a solid phase support through a linker entity.

38. A method according to claim 37 wherein the linker entity is an acid labile linker, selected from chlorotritylchloride, Wang, Rink, Sieber or related linkers.

39. A method according to claim 38 wherein the solid phase support is selected from the group comprising polystyrene, modified polystyrene and PEGA.

40. A method for the preparation of a dendrimer conjugate according to any of claims 1-17 in which the dendrimer conjugate contains surface sulfonylurea (sulfamide) groups, the method comprising the reaction of dendrimer with a sulfonylamide (>SO₂NHR) reagent, such as e.g. chlorosulfonyl-isocyanate, halo-sulfamide, chlorsulfonyl-*tert*-butylsulfamate or other sulfonylamide reagent.

41. A method for the preparation of a dendrimer conjugate according to any of claims 1-17 in which the dendrimer conjugate contains surface guanidine groups, the method comprising the reaction of dendrimer with di-boc-S-methylisothiourea, di-Boc-thiourea or condensing agents such as carbodiimides, phosphonium salts or other condensing reagents.

42. A method for the preparation of a dendrimer conjugate according to any of claims 1-17 in which the dendrimer conjugate contains surface thiourea groups, the method comprising the reaction of dendrimer with thiocarbamoyl (-(C=S)NHR) reagents, such as e.g. alkyl-thiocarbamoyl halides or other thiocarbamoyl reagents.

43. A method for the preparation of a dendrimer conjugate according to any of claims 1-17 in which the dendrimer conjugate contains surface urea groups, the method comprising the reaction of dendrimer with carbamoyl (-(C=O)NHR) reagents such as e.g. alkyl-carbamoyl halides or other carbamoyl reagents.

## Patentansprüche

1. Zwischen einem Dendrimer und einer proteinsolubilisierenden Substanz gebildetes Dendrimerkonjugat, aufweisend die Struktur
D(R)n ,
wobei
D für das Dendrimer steht;
R für ein Radikal der proteinsolubilisierenden Substanz steht, die gleich oder verschieden sein kann, und ausgewählt aus 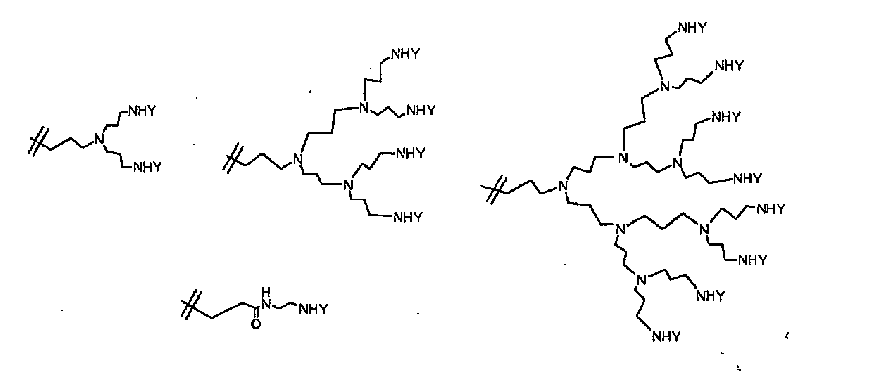 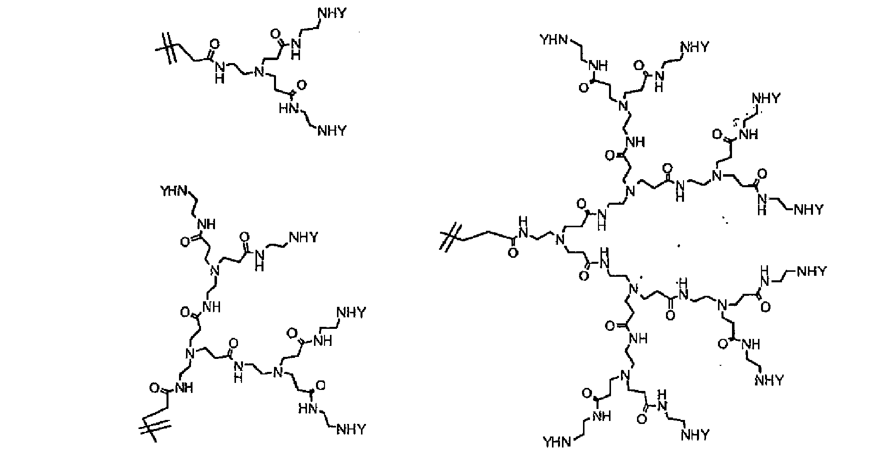 ist,
wobei Y aus der Gruppe ausgewählt ist, bestehend aus 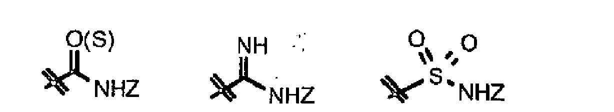 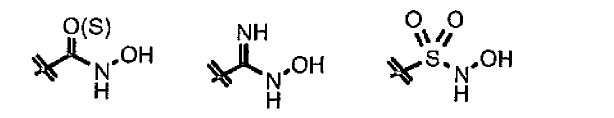 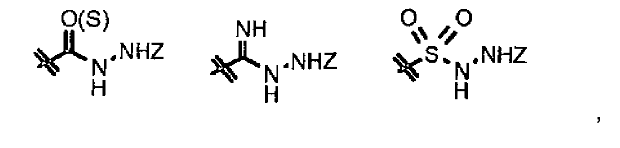 wobei Z aus der Gruppe ausgewählt ist, bestehend aus 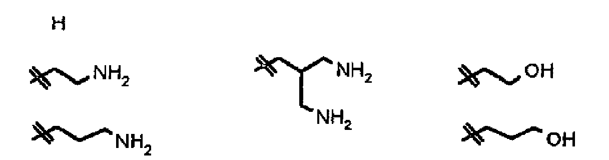 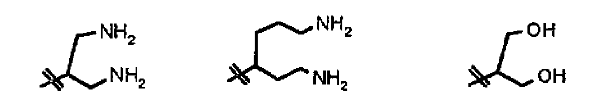 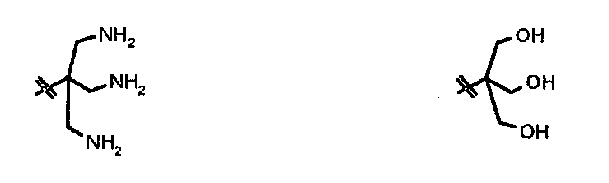 und, n eine ganze Zahl ist, die größer ist als 1;
welche proteinsolubilisierende Substanz eine im Dendrimer nicht gefundene Struktur aufweist, und welches Konjugat - durch Behandlung von Proteinaggregaten mit dem Dendrimerkonjugat - eine Erhöhung um einen Faktor von mehr als 1 bei der Solubilisierung von Proteinaggregaten gegenüber dem Erreichten durch Behandlung von Proteinaggregaten unter den gleichen Behandlungsbedingungen mit einer physikalischen Mischung des Dendrimers und der proteinsolubilisierenden Substanz bewirkt, wobei die physikalische Mischung das gleiche Molverhältnis der proteinsolubilisierenden Substanz zum Dendrimer enthält als das im Dendrimerkonjugat.

2. Dendrimerkonjugat nach Anspruch 1, wobei das Dendrimer kovalent an eine oder mehrere gleiche oder verschiedene proteinsolubilisierende Substanzen gebunden ist.

3. Dendrimerkonjugat nach irgendeinem der Ansprüche 1-2, wobei die proteinsolubilisierende Substanz ein Proteindenaturierungsmittel darstellt.

4. Dendrimerkonjugat nach Anspruch 3, wobei das Proteindenaturierungsmittel aus der aus Harnstoffen, Thioharnstoffen, Sulfonylharnstoffen, Semicarbaziden, Hydraziden, Thiosemicarbaziden, Guanidinen und Chaotropen bestehenden Gruppe ausgewählt ist.

5. Dendrimerkonjugat nach irgendeinem der Ansprüche 1-4, wobei die Solubilisierung der Proteinaggregate um einen Faktor von mindestens 1,5 oder mindestens 2 erhöht ist.

6. Dendrimerkonjugat nach irgendeinem der Ansprüche 1-5, enthaltend eine oder mehrere Oberflächengruppen, die nicht mit einer proteinsolubilisierenden Substanz belegt sind.

7. Dendrimerkonjugat nach irgendeinem der Ansprüche 1-6, wobei das Dendrimer (D) ein mehrwertiges funktionales Dendrimer darstellt, welches eine dendritische Struktur aufweist, die sich von einem oder mehreren Kernpunkten durch multiple Generationen von aufeinanderfolgenden Schichten erstreckt, wobei jede Schicht einen oder mehrere Verzweigungspunkte aufweist, um in Oberflächengruppen zu enden.

8. Dendrimerkonjugat nach Anspruch 7, wobei das Dendrimer (D) durch die Formel:
X-(V)ₐ-(W)_{b}
dargestellt ist,
wobei X ein einen oder mehrere Verzweigungspunkte aufweisendes multifunktionales Segment darstellt,
V eine Linker- oder Spacergruppe darstellt, die verzweigt oder linear sein können,
W eine Oberflächengruppe darstellt, und
a sowie b ganze Zahlen sind, so dass jede Linkergruppe V in einer oder mehreren Oberflächengruppen W terminiert.

9. Dendrimerkonjugat nach irgendeinem der Ansprüche 7 oder 8, wobei das Dendrimer globulär oder baumförmig ist.

10. Dendrimerkonjugat nach irgendeinem der Ansprüche 7-9, wobei die Generation des Dendrimers im Bereich von 0 bis 20 liegt.

11. Dendrimerkonjugat nach irgendeinem der Ansprüche 7-10, wobei die Molekularmasse des unmodifizierten Dendrimers vom 50 bis 30000 liegt.

12. Dendrimerkonjugat nach irgendeinem der Ansprüche 7-11, wobei die Anzahl von Oberflächengruppen des Dendrimers zwischen 2 und 256 liegt.

13. Dendrimerkonjugat nach irgendeinem der Ansprüche 7-12, wobei die Oberflächengruppen des Dendrimers (D) Aminfunktionalitäten darstellen.

14. Dendrimerkonjugat nach irgendeinem der Ansprüche 7-13, wobei das Dendrimer ein PPI-Dendrimer oder ein PEI-Dendrimer oder en PAMAM-Dendrimer darstellt.

15. Dendrimerkonjugat nach irgendeinem der Ansprüche 7-14, wobei das Dendrimer eine der folgenden Kernstrukturen aufweist 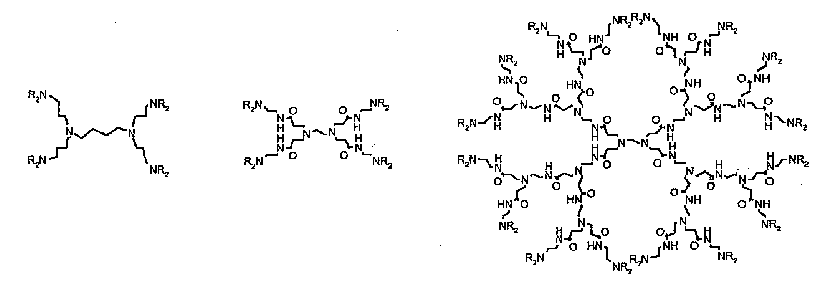 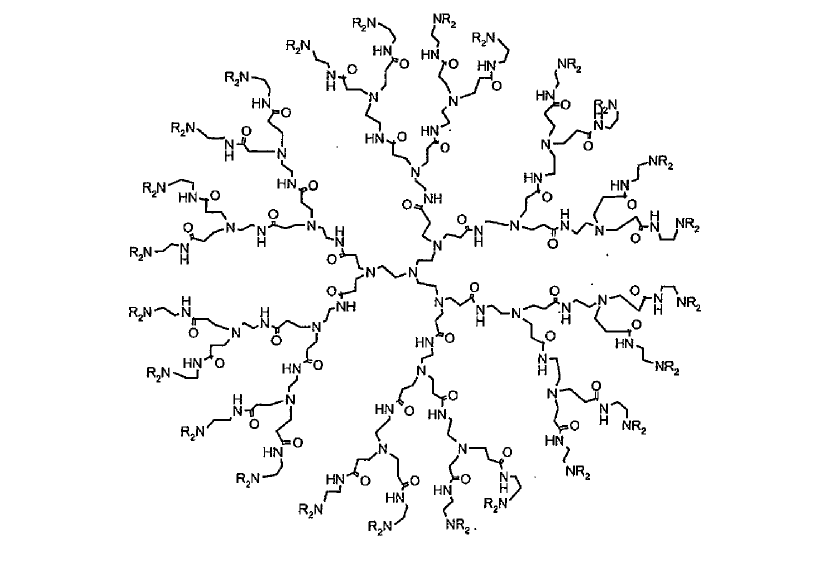 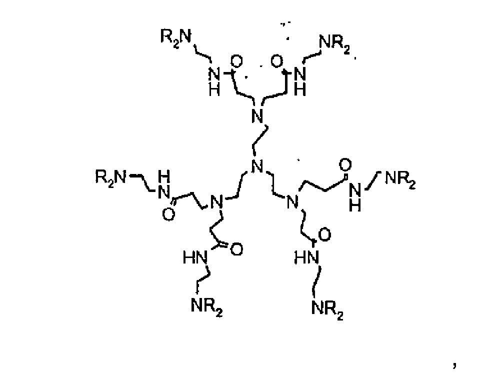 wobei R dieselbe Bedeutung wie in Anspruch 1 hat.

16. Dendrimerkonjugat nach irgendeinem der Ansprüche 1-6, wobei das Dendrimer (D) ein Konjugat von zwei oder mehreren mehrwertigen funktionalen Dendrimeren wie in den Ansprüchen 7-15 definiert darstellt.

17. Dendrimerkonjugat nach irgendeinem der vorhergehenden Ansprüche, welches Dendrimerkonjugat einen EC50-Wert von 10-500 µg/ml aufweist.

18. Verwendung eines Dendrimerkonjugats nach irgendeinem der Ansprüche 1-17 zur Herstellung eines Medikaments zur Behandlung von proteinaggregatbedingten Krankheiten.

19. Verwendung nach Anspruch 18, wobei die proteinaggregatbedingte Krankheit aus der aus Alzheimer-Krankheit, Creutzfeldt-Jakob-Krankheit, varianter Creutzfeldt-Jakob-Krankheit, letaler familiärer Insomnie, Gerstmann-Sträussler-Scheinker-Syndrom, PrP-zerebraler Amyloidangiopathie, Scrapie, Bovine Spongiforme Enzephalopathie, chronisch zehrender Krankheit, transmissibler Nerzenzephalopathie, Pick-Krankheit, Parkinson-Krankheit, amyotrophischer Lateralsklerose, frontotemporaler Demenz, Diabetes Typ 2, multipler Myelomplasmazelldyskrasie, familiärer Amyloidpolyneuropathie, Medullärem Schilddrüsenkarzinom, chronischem Nierenversagen, kongestivem Herzversagen, seniler kardialer und systemischer Amyloidose, chronischer Inflammation, Atherosklerose, familiärer Amyloidose und Huntington-Krankheit bestehenden Gruppe ausgewählt ist.

20. Verwendung nach irgendeinem der Ansprüche 18 oder 19, wobei das Protein des Proteinaggregats aus der aus APP, Aß-Peptid, α1-Antichymotrypsin, Tau, Nicht-Aß-Komponent, Presenillin 1, Presenillin 2, apoE, Prionprotein, hierunter proteaseresistentem Prionprotein, SOD, Pick body, α-Synuclein, Anylin, IgGL-Kette, Transthyretin, Procalcitonin, β2-Mikroglobulin, atrialem natriuretischem Faktor, Serumamyloid A, ApoA1, Gelsolin und Huntington-Krankheit bestehenden Gruppe ausgewählt ist.

21. Verwendung nach irgendeinem der Ansprüche 18 oder 19, wobei die proteinaggregatbedingte Krankheit eine prionbedingte Krankheit ist.

22. Verwendung nach irgendeinem der Ansprüche 18 oder 19, wobei die proteinaggregatbedingte Krankheit eine amyloidbedingte Krankheit ist.

23. Verwendung von Dendrimerkonjugaten nach irgendeinem der Ansprüche 1-17 zur Reduktion der Infektiosität von Prionproteinen in vitro.

24. Verwendung von Dendrimerkonjugaten nach irgendeinem der Ansprüche 1-17 bei Desinfizierung von mit Proteinaggregaten kontaminiertem Material.

25. Verwendung nach Anspruch 24, wobei die Proteinaggregate Prionproteinaggregate darstellen.

26. Verfahren zur Identifizierung und/oder Klassifizierung von Proteinaggregaten in einem Säuger, welches Verfahren die folgenden Schritte umfasst:
a) Behandlung der Proteinaggregate mit einem Dendrimerkonjugat wie in den Ansprüchen 1-17 definiert
b) Analyse eines oder mehrerer Produkte des Schritts a).

27. Verfahren nach Anspruch 26, wobei Schritt b) die folgenden Schritte umfasst
I. Inkubation des behandelten Proteinaggregats aus Schritt a) mit einem breiten Spektrum an Protease wie z.B. Proteinase K
II. Detektion verbleibender Proteinaggregate durch einen oder mehrere Prozesse, die aus der Gruppe ausgewählt sind, umfassend SDS-PAGE und Immunblot mit proteinspezifischen Antikörpern, ELISA, Immunelektrophorese und Immunhistochemie.

28. Verfahren nach Anspruch 26, wobei Schritt b) eine Inkubation der behandelten Proteinaggregate aus Schritt a) mit einem Antikörper umfasst, welcher empfindlich auf Veränderungen in der Struktur eines im Proteinaggregat vorhandenen Proteins reagiert.

29. Verfahren nach Anspruch 26, welches Verfahren zusätzlich den Schritt der weiteren Behandlung der behandelten Proteinaggregate aus Schritt a) mit einem Proteindenaturierungsmittel wie z.B. Harnstoff zwischen den Schritten a) und b) umfasst.

30. Verfahren nach Anspruch 26, welches Verfahren zusätzlich die folgenden Schritte umfasst
i) Wiederholen der Schritte a) und b) mit einem anderen Dendrimerkonjugat und
ii) wahlweise Vergleichen von Ergebnissen aus den Dendrimerkonjugaten, um Informationen über die Herkunft der Proteinaggregate zu erhalten.

31. Verwendung von Dendrimerkonjugaten nach irgendeinem der Ansprüche 1-17 zur Identifizierung von Prionproteinaggregaten.

32. Verwendung von Dendrimerkonjugaten nach irgendeinem der Ansprüche 1-17 zur Klassifizierung der Proteinaggregate in spezifische Stämme nach ihrer Suszeptibilität gegenüber dem in Anspruch 26 beschriebenen Verfahren.

33. Verwendung nach Anspruch 32, wobei die Proteinaggregate Prionproteinaggregate darstellen.

34. Verfahren zur Desinfizierung eines Objekts, welches Verfahren ein Kontaktieren des Objekts mit einer Zusammensetzung umfasst, welche ein Dendrimerkonjugat nach irgendeinem der Ansprüche 1-17 enthält.

35. Verfahren zur Entfernung von Proteinaggregaten aus einer von einem Tier stammenden Nahrung, welches Verfahren ein Kontaktieren der Nahrung mit einer Zusammensetzung umfasst, die ein Dendrimerkonjugat nach irgendeinem der Ansprüche 1-17 enthält.

36. Verwendung von Dendrimerkonjugaten nach irgendeinem der Ansprüche 1-17 bei der Zubereitung eines Medikaments zur Verwendung bei der Prophylaxe und/oder Diagnose von Proteinaggregatbedingten Krankheiten.

37. Verfahren zur Zubereitung eines Dendrimerkonjugats nach irgendeinem der Ansprüche 1-17, wobei die Zubereitung durchgeführt wird, während das Dendrimer (D) auf einen Festphasenträger durch eine Linkereinheit gepfropft ist.

38. Verfahren nach Anspruch 37, wobei die Linkereinheit einen aus Chlortritylchlorid, Wang, Rink, Sieber oder zugehörigen Linkern ausgewählten säurelabilen Linker darstellt.

39. Verfahren nach Anspruch 38, wobei der Festphasenträger aus der Gruppe ausgewählt wird, die Polystyrol, modifiziertes Polystyrol und PEGA umfasst.

40. Verfahren zur Zubereitung eines Dendrimerkonjugats nach irgendeinem der Ansprüche 1-17, wobei das Dendrimerkonjugat Oberflächensulfonylharnstoff(sulfamid)gruppen enthält, welches Verfahren die Reaktion des Dendrimers mit einem Sulfonylamid(>SO₂NHR)-Reagenz, wie z.B. Chlorsulfonylisocyanat-, Halogensulfamid-, Chlorsulfonyl-*Tert*-Butylsulfamat- oder einem anderen Sulfonylamidreagenz, umfasst.

41. Verfahren zur Zubereitung eines Dendrimerkonjugats nach irgendeinem der Ansprüche 1-17, wobei das Dendrimerkonjugat Oberflächenguanidingruppen enthält, welches Verfahren die Reaktion des Dendrimers mit einem Di-Boc-S-Methylisothioharnstoff, Di-Boc-Thioharnstoff oder Kondensationsmitteln wie Carbodiimiden, Phosphoniumsalzen oder anderen Kondensationsreagenzien umfasst.

42. Verfahren zur Zubereitung eines Dendrimerkonjugats nach irgendeinem der Ansprüche 1-17, wobei das Dendrimerkonjugat Oberflächenthioharnstoffgruppen enthält, welches Verfahren die Reaktion des Dendrimers mit Thiocarbamoyl(-(C=S)NHR)-Reagenzien, wie z.B. Alkylthiocarbamoylhaliden oder anderen Thiocarbamoylreagenzien, umfasst.

43. Verfahren zur Zubereitung eines Dendrimerkonjugats nach irgendeinem der Ansprüche 1-17, wobei das Dendrimerkonjugat Oberflächenharnstoffgruppen enthält, welches Verfahren die Reaktion des Dendrimers mit Carbamoyl(-(C=O)NHR)-Reagenzien, wie z.B. Alkyl-Carbamoylhaliden oder anderen Carbamoylreagenzien, umfasst.

## Revendications

1. Conjugué de dendrimère formé entre un dendrimère et une substance dissolvant les protéines, présentant la structure
D(R)n
dans laquelle
D est le dendrimère
R est un radical de la substance dissolvant les protéines qui peut être la même ou une autre substance choisie parmi 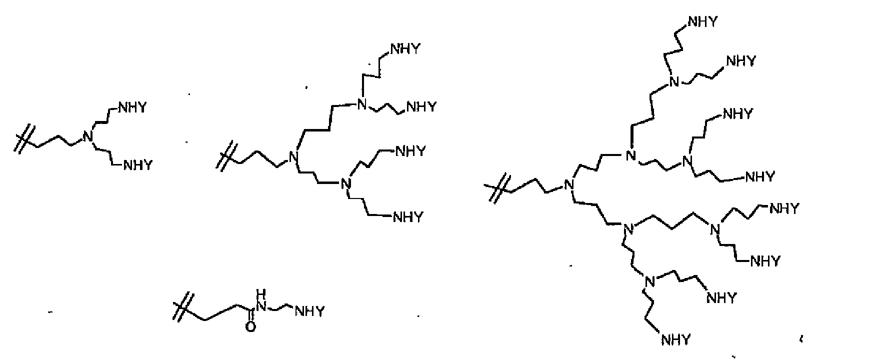 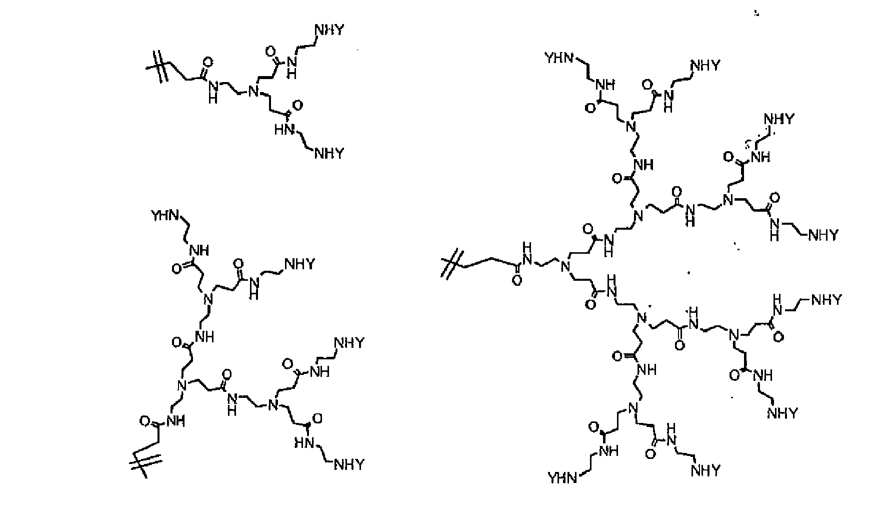 où Y est sélectionné parmi le groupe constitué par 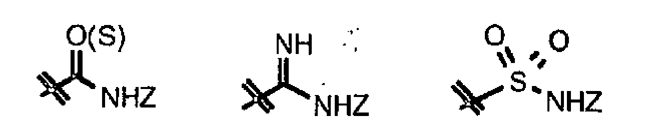 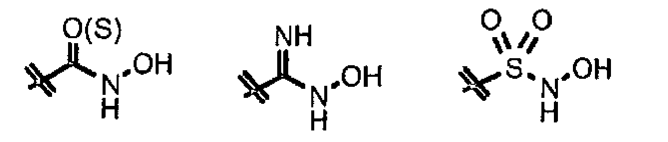 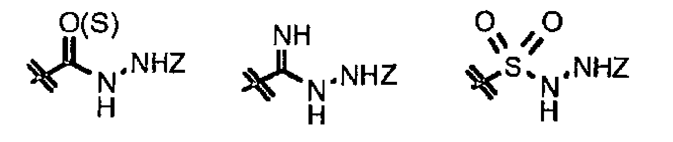 où Z est sélectionné parmi le groupe constitué par 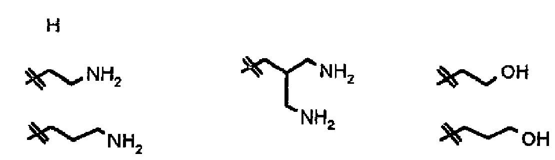 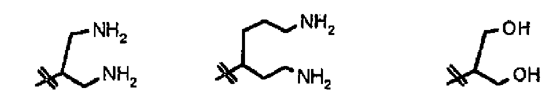 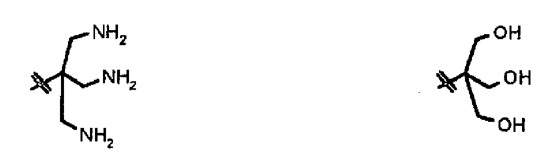 et n est un entier supérieur à 1 ;
dite substance dissolvant les protéines présentant une structure que l'on ne trouve pas dans le dendrimère, et le conjugué - après traitement d'agrégats de protéines avec le conjugué de dendrimère - causant une augmentation par un facteur supérieur à 1 dans la solubilité d'agrégats de protéines au-dessus de celle obtenue après traitement d'agrégats de protéines dans les mêmes conditions de traitement avec un mélange physique du dendrimère et la substance dissolvant les protéines, le mélange physique comprenant le même ratio molaire de la substance dissolvant les protéines par rapport au dendrimère que celui dans le conjugué de dendrimère.

2. Conjugué de dendrimère selon la revendication 1, dans lequel le dendrimere est lié de manière covalente à l'une ou à plusieurs ou à différentes substances dissolvant les protéines.

3. Conjugué de dendrimère selon l'une quelconque des revendications 1 à 2, dans lequel la substance dissolvant les protéines est une protéine dénaturée.

4. Conjugué de dendrimère selon la revendication 3, dans lequel la protéine dénaturée est sélectionnée parmi le groupe constitué par urées, thiourées, sulfonylurées, semicarbazides, hydrazides, thiosemicarbazides, guanidines et chaotropes.

5. Conjugué de dendrimère selon l'une quelconque des revendications 1 à 4, dans lequel la solubilité des agrégats de protéines est augmentée par un facteur d'au moins 1,5 ou au moins 2.

6. Conjugué de dendrimère selon l'une quelconque des revendications 1 à 5, comprenant un ou plusieurs groupes de surface qui ne sont pas occupés par une substance dissolvant les protéines.

7. Conjugué de dendrimère selon l'une quelconque des revendications 1 à 6, dans lequel le dendrimère (D) est un dendrimère fonctionnel et multivalent présentant une structure dendritique qui s'étend à partir de l'un ou plusieurs points centraux à travers de multiples générations de couches successives, dont chaque couche possède un ou plusieurs points d'embranchement, pour finir dans les groupes de surface.

8. Conjugué de dendrimère selon la revendication 7, dans lequel le dendrimère (D) est représenté par la formule :
X-(V)ₐ-(W)_{b}
dans laquelle X est un segment multifonctionnel présentant un ou plusieurs points d'embranchement,
V est un groupe de liaison ou d'espacement qui peut être ramifiée ou linéaire,
W est un groupe de surface, et
a et b sont des entiers si bien que chaque groupe de liaison V se termine dans l'un ou plusieurs groupes de surface W.

9. Conjugué de dendrimère selon l'une quelconque des revendications 7 ou 8, dans lequel le dendrimère est globulaire ou arboriforme.

10. Conjugué de dendrimère selon l'une quelconque des revendications 7 à 9, dans lequel la génération du dendrimère est comprise entre 0 et 20.

11. Conjugué de dendrimère selon l'une quelconque des revendications 7 à 10, dans lequel la masse moléculaire du dendrimère non modifié est comprise entre 50 et 30000.

12. Conjugué de dendrimère selon l'une quelconque des revendications 7 à 11, dans lequel le nombre de groupes de surface du dendrimère est compris entre 2 et 256.

13. Conjugué de dendrimère selon l'une quelconque des revendications 7 à 12, dans lequel les groupes de surface du dendrimère (D) sont des fonctionnalités amines.

14. Conjugué de dendrimère selon l'une quelconque des revendications 7 à 13, dans lequel le dendrimère est un dendrimère PPI ou un dendrimère PEI ou un dendrimère PAMAM.

15. Conjugué de dendrimère selon l'une quelconque des revendications 7 à 14, dans lequel le dendrimère présente l'une des structures centrales suivantes : 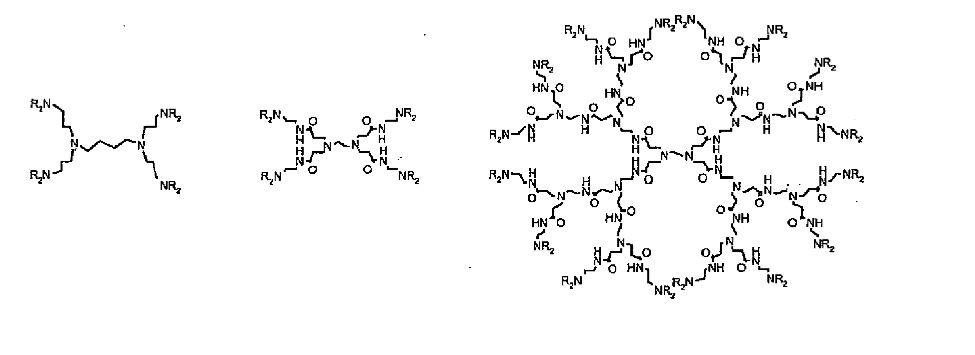 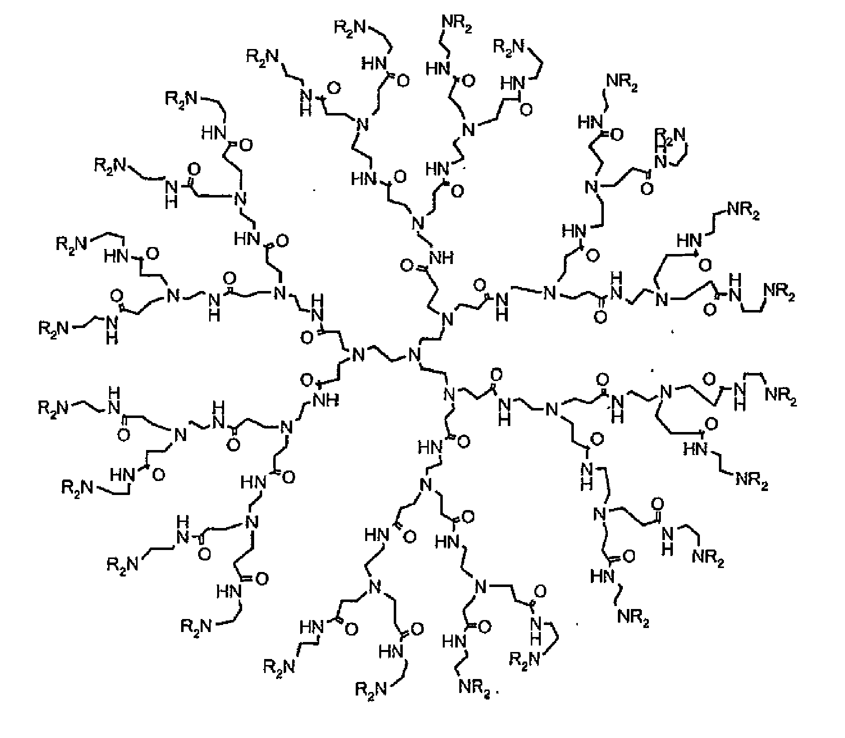 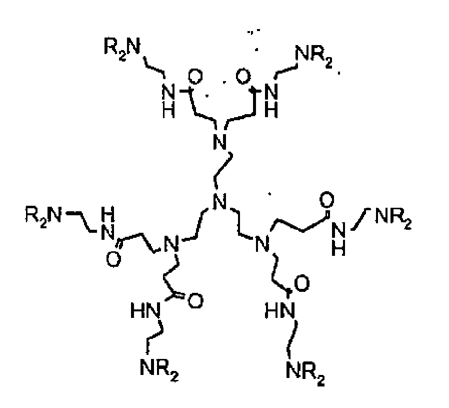 où R présente la même signification que celle de la revendication 1.

16. Conjugué de dendrimère selon l'une quelconque des revendications 1 à 6, dans lequel le dendrimère (D) est un conjugué de deux ou plusieurs dendrimères fonctionnels et multivalents tels que définis dans les revendications 7 à 15.

17. Conjugué de dendrimère selon l'une quelconque des revendications précédentes, qui présente une valeur EC50 de 10-500µg/ml.

18. Utilisation d'un conjugué de dendrimère selon l'une quelconque des revendications 1 à 17 pour la préparation d'un médicament destiné au traitement des maladies liées à l'agrégation de protéines.

19. Utilisation selon la revendication 18, dans laquelle la maladie liée à l'agrégation de protéines est sélectionnée parmi le groupe constitué par la maladie d'Alzheimer, la maladie de Creutzfeldt-Jakob, la variante de la maladie de Creutzfeldt-Jakob, l'insomnie fatale familiale, le syndrome de Gerstmann-Straussler-Scheinker, l'angiopathie amyloïde cérébrale à protéine prion (PrP), la scrapie, l'encéphalopathie spongiforme bovine, la maladie du dépérissement chronique, l'encéphalopathie transmissible de vison, la maladie de Pick, la maladie de Parkinson, la sclérose latérale amyotrophique, la démence fronto-temporale, le diabète de type 2, le myélome multiple - la dyscrasie de cellules de plasma, la polyneuropathie amyloïde familiale, le carcinome médullaire de la thyroïde, l'insuffisance rénale chronique, l'insuffisance cardiaque congestive, l'amyloïdose cardiaque et systémique sénile, l'inflammation chronique, l'athérosclérose, l'amyloïdose familiale et la maladie de Huntington.

20. Utilisation selon l'une quelconque des revendications 18 ou 19, dans laquelle la protéine de l'agrégat de protéines est sélectionnée parmi le groupe constitué par la protéine APP, le peptide Aβ, l'α1-antichymotrypsine, tau, composant non-Aβ, la préséniline 1, la préséniline 2, apo E, la protéine prion y compris la protéine prion résistante à la protéase, SOD, les corps de Pick, l'α-synucléine, l'aniline, la chaîne d'IgGL, la thransthyrétine, la procalcitonine, la β2-microglobuline, le facteur natriurétique auriculaire, le sérum amyloïde A, apo A1, la gelsoline et Huntington.

21. Utilisation selon l'une des revendications 18 ou 19, dans laquelle la maladie liée à l'agrégation de protéines est une maladie à prions.

22. Utilisation selon l'une des revendications 18 ou 19, dans laquelle la maladie liée à l'agrégation de protéines est une maladie amyloïde.

23. Utilisation de conjugués de dendrimère selon l'une quelconque des revendications 1 à 17 visant à réduire l'infectivité de protéines prions in vitro.

24. Utilisation de conjugués de dendrimère selon l'une quelconque des revendications 1 à 17 dans la désinfection de matériaux étant contaminés par des agrégats de protéines.

25. Utilisation selon la revendication 24, dans laquelle les agrégats de protéines sont des agrégats de protéines prions.

26. Procédé d'identification et/ou de classement des agrégats de protéines dans un mammifère, le procédé comprenant les étapes de :
a) traitement des agrégats de protéines par un conjugué de dendrimère comme défini dans les revendications 1 à 17,
b) analyse d'un ou plusieurs produits de l'étape a)

27. Procédé selon la revendication 26, dans lequel l'étape b) comprend les étapes de :
I. l'incubation des agrégats de protéines traités dans l'étape a) avec une protéase à spectre large, telle que *p. ex.* protéinase K
II. la détection des agrégats restants de protéines par l'un ou plusieurs procédés sélectionnés parmi le groupe comprenant : SDS-PAGE et l'immunoblotting avec des anticorps spécifiques de protéine, ELISA, l'immunoélectrophorèse et l'immunohistochimie.

28. Procédé selon la revendication 26, dans lequel l'étape b) comprend l'incubation des agrégats de protéines traités dans l'étape a) avec un anticorps sensible aux changements de la structure d'une protéine présente dans l'agrégat de protéines.

29. Procédé selon la revendication 26, en outre comprenant l'étape d'un traitement supplémentaire des agrégats de protéines traités dans l'étape a) par une protéine dénaturée, telle que *p. ex.* l'urée entre les étapes a) et b).

30. Procédé selon la revendication 26, en outre comprenant l'étape de
i) répéter les étapes a) et b) avec un autre conjugué de dendrimère, et
ii) éventuellement comparer les résultats des conjugués de dendrimère afin d'obtenir des renseignements sur l'origine des agrégats de protéines.

31. Utilisation des conjugués de dendrimère selon l'une quelconques des revendications 1 à 17 pour l'identification des agrégats de protéines prions.

32. Utilisation des conjugués de dendrimère selon l'une quelconques des revendications 1 à 17 pour le classement des agrégats de protéines en souches spécifiques selon leur susceptibilité au procédé décrit dans la revendication 26.

33. Utilisation selon la revendication 32, dans laquelle les agrégats de protéines sont des agrégats de protéines prions.

34. Procédé pour la désinfection d'un objet, le procédé comprenant la mise en contact de l'objet avec une composition comprenant un conjugué de dendrimère selon l'une des revendications 1 à 17.

35. Procédé visant à enlever les agrégats de protéines de denrées alimentaires d'origine animale, le procédé comprenant la mise en contact des denrées alimentaires avec une composition contenant un conjugué de dendrimère selon l'une quelconque des revendications 1 à 17.

36. Utilisation des conjugués de dendrimère selon l'une quelconque des revendications 1 à 17 dans la préparation d'un médicament à utiliser dans la prophylaxie et/ou le diagnostic des maladies liées à l'agrégation de protéines.

37. Procédé pour la préparation d'un conjugué de dendrimère selon l'une quelconque des revendications 1 à 17, dans lequel la préparation est réalisée pendant que le dendrimère (D) est greffé à une phase de support solide par une entité de liaison.

38. Procédé selon la revendication 37, dans lequel l'entité de liaison est une séquence de liaison labile d'acide, sélectionnée parmi chlorotritylchloride, Wang, Rink, Sieber ou d'autres séquences de liaison associées.

39. Procédé selon la revendication 38, dans lequel la phase de support solide est sélectionnée parmi le groupe constitué par le polystyrène, le polystyrène modifié et PEGA.

40. Procédé pour la préparation d'un conjugué de dendrimère selon l'une quelconque des revendications 1 à 17, dans lequel le conjugué de dendrimère comprend des groupes de surface de la sulfonylurée (le sulfamide), le procédé comprenant la réaction de dendrimère avec un réactif de sulfonylamide (>SO₂NHR), tel que p. ex. l'isocyanate de chlorosulfornyle, halosulfamide, chlorosulfonyle *tert*-butylsulfamate ou d'autres réactifs de sulfonylamide.

41. Procédé pour la préparation d'un conjugué de dendrimère selon l'une quelconque des revendications 1 à 17, dans lequel le conjugué de dendrimère comprend des groupes de surface de la guanidine, le procédé comprenant la réaction de dendrimère avec di-boc-S-methylisothiourée, di-Boc thiourée ou des agents condensateurs, tels que carbodiimides, sels de phosphonium ou d'autres agents condensateurs.

42. Procédé pour la préparation d'un conjugué de dendrimère selon l'une quelconque des revendications 1 à 17, dans lequel le conjugué de dendrimère comprend des groupes de surface de la thiourée, le procédé comprenant la réaction de dendrimère avec les réactifs de thiocarbamoyle (-(C=S)NHR), tels que p. ex. des halogénures d'alkyl-thiocarbamoyle ou d'autres réactifs de thiocarbamoyle.

43. Procédé pour la préparation d'un conjugué de dendrimère selon l'une quelconque des revendications 1 à 17, dans lequel le conjugué de dendrimère comprend des groupes de surface de l'urée, le procédé comprenant la réaction de dendrimère avec les réactifs de carbamoyle (-(C=O)NHR), tels ue p. ex. des halogénures d'alkyl-carbamoyle ou d'autres réactifs de carbamoyle.
